# EUROPÄISCHE PATENTSCHRIFT

(11) **EP 0 755 918 B1**
(45) Veröffentlichungstag und Bekanntmachung des Hinweises auf die Patenterteilung: **06.09.2000**
(21) Anmeldenummer: 96111676.1
(22) Anmeldetag: 19.07.1996
(51) Int. Cl.: C07C 255/55, C09K 19/20, C07C 69/90, C07D 239/34, C07D 213/30, C07C 43/20, C09K 19/30, C09K 19/34

(54) **Photovernetzbare flüssigkristalline 1,2-Phenylen-Derivate**
Photocrosslinkable liquid crystalline 1,2-phenylene derivatives
Dérivés photoréticulables du 1,2-phénylène ayant des propriétés de cristaux liquides

(30) Priorität: 28.07.1995 CH 222195; 09.05.1996 EP 96107341
(43) Veröffentlichungstag der Anmeldung: 29.01.1997
(73) Patentinhaber: Rolic AG, 6301 Zug (CH)
(72) Erfinder: Kelly, Stephen, Beverley HU17 8XA, East Yorkshire (GB)
(74) Vertreter: England, Christopher David

(56) Entgegenhaltungen:
- EP-A- 0 611 981
- EP-A- 0 700 981
- WO-A-95/16007
- WO-A-95/24455
- DE-A- 4 226 994

## Beschreibung

Die vorliegende Erfindung betrifft photovernetzbare flüssigkristalline 1,2-Phenylen-Derivate, flüssigkristalline Gemische, die solche Verbindungen enthalten, sowie deren Verwendung im vernetzten Zustand als optische Bauelemente.

Durch geeignete Orientierungsschichten oder in einem Feld können photovernetzbare Flüssigkristalle, welche mit einer geeigneten Menge eines Photoinitiators versehen sind, auf einem Substrat oder in einer Zelle orientiert werden und dann in diesem Zustand durch Bestrahlen mit Licht einer geeigneten Wellenlänge vernetzt werden. Die dadurch erzeugte Struktur bleibt auch bei hohen Temperaturen erhalten. So lassen sich optische Bauelemente, wie zum Beispiel Wellenleiter, optische Gitter und Filter, piezoelektrische Zellen und Zellen mit nicht-linearen optischen (NLO) Eigenschaften, usw. herstellen. Solche optische Bauelemente können zum Beispiel für Frequenzverdoppelung (SHG) oder in Farbfilter verwendet werden.

Weitere Eigenschaften, wie beispielsweise die Doppelbrechung, der Brechungsindex, die Transparenz, usw., müssen je nach Anwendungsgebiet unterschiedlichen Anforderungen genügen. Beispielsweise sollten Materialien für optische Filter eine starke Absorption in einer Richtung senkrecht zur Filteroberfläche aufweisen.

Neben dem generellen Interesse an photovernetzbaren Flüssigkristallen für optische Bauelemente, eignen sich solche flüssigkristalline Materialien als Cladding von Glasfibern für optische Datenübertragung. Der Einsatz solcher Materialien erhöht den elastischen Modulus in der Längsachse der Fiber, verkleinert die thermischen Expansionskoeffizienten und vermindert so Mikroverbiegungsverluste. Dies führt zu einer erhöhten mechanischen Stabilität.

Die photovernetzbaren Flüssigkristalle müssen eine gute chemische und thermische Stabilität, gute Löslichkeit in gängigen Lösungsmitteln und eine gute Stabilität gegenüber elektrischen Feldern und elektromagnetischer Strahlung aufweisen. Sie sollten in einem Temperaturbereich von etwa 25°C bis etwa +100°C, insbesondere von etwa 25°C bis etwa +80°C, eine geeignete Mesophase besitzen.

Da Flüssigkristalle in der Regel als Mischungen mehrerer Komponenten zur Anwendung gelangen, ist es wichtig, dass die Komponenten untereinander gut mischbar sind. Herkömmliche photochemisch oligomerisierbare oder polymerisierbare Flüssigkristalle besitzen in der Regel einen hohen Schmelz- und Klärpunkt. Dies hat den Nachteil, dass bei der Verarbeitung vorzeitig eine spontane, thermische Polymerisation eintreten kann. Die spontane, thermische Polymerisation kann eintreten, weil die Verarbeitung bei Temperaturen knapp unter dem Klärpunkt durchgeführt wird, da bei dieser Temperatur die Viskosität im flüssigkristallinen Zustand am niedrigsten und daher günstig für eine gute Orientierbarkeit ist. Diese spontane Polymerisation führt zu Domänenbildung, wodurch die optischen und thermischen Eigenschaften in den erzeugten, vernetzten Schichten deutlich beeinträchtigt werden. Der Schmelzpunkt kann durch die Herstellung komplizierter Mischungen mit mehreren Komponenten herabgesetzt werden, was zwar eine Verarbeitung bei niedrigeren Temperaturen erlaubt, aber die Gefahr einer Kristallisation der herkömmlichen polymerisierbaren Flüssigkristalle mit sich bringt. Photochemisch oligomerisierbare oder polymerisierbare Verbindungen werden beispielsweise in EP-A-0 331 233 beschrieben.

Es stellte sich somit die Aufgabe, für die Anwendung in optischen Bauteilen, optischen Filtern, Kanalwellenleitern, Mach-Zehnderstrukturen, homeotropen Schichten, usw. photochemisch oligomerisierbare oder polymerisierbare Verbindungen bereitzustellen, welche sich durch eine besonders hohe optische Anisotropie Δn auszeichnen. Sie sollten niedrigere Schmelz- und Klärpunkte besitzen, damit die Viskosität bei den normalen Verarbeitungstemperaturen im flüssigkristallinen Zustand nicht zu hoch ist. Weiter sollten sie möglichst domänenfrei orientierbar und strukturierbar sein und auch eine ausgezeichnete thermische Stabilität und Langzeitstabilität im vernetzten Zustand aufweisen. Zudem sollten sie, insbesondere für Kanalwellenleiter, Mach-Zehnderstrukturen, homeotrope Schichten, usw., eine positive dielektische Anisotropie aufweisen und sich in einem elektrischen Feld orientieren lassen. Dies erlaubt, zum Beispiel, die zusätzliche Strukturierung durch Verwendung von Elektroden von photochemisch oligomerisierbaren oder polymerisierbaren Verbindungen, welche bereits durch eine Orientierungsschicht homogen orientiert sind. Herkömmliche photochemisch oligomerisierbare oder polymerisierbare Flüssigkristalle besitzen in der Regel eine negative oder eine sehr schwach positive dielektische Anisotropie.

Die vorliegende Erfindung stellt nun Verbindungen, die in hervorragender Weise als Einzelkomponenten oder als Komponenten von Flüssigkristall-Mischungen für die obengenannten Anwendungen geeignet sind, zur Verfügung. Gegenstand der vorliegenden Erfindung sind Verbindungen der allgemeinen Formel I 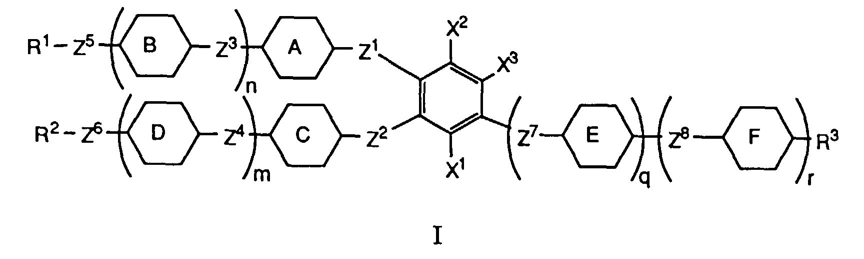 worin
- Ringe A - F: je Pyridin-2,5-diyl, Pyrimidin-2,5-diyl, trans-1,4-Cyclo-hexylen, trans-1,3-Dioxan-2,5-diyl oder unabhängig voneinander gegebenenfalls mit Halogen, Methyl und/oder Cyano substituiertes 1,4-Phenylen;
- Z¹, Z² und Z⁷: unabhängig voneinander ⁻CH₂-(CH₂)ₛ-, -(CH₂)ₛO-, -O(CH₂)ₛ-, -COO-, -OOC-, -(CH₂)ₛCOO- ⁻ oder -(CH₂)ₛOOC-;
- Z³, Z⁴ und Z⁸: unabhängig voneinander eine Einfachbindung, -CH₂CH₂-, -CH₂O-, -OCH₂-, -COO-, -OOC-, -(CH₂)₄-, -O(CH₂)₃- oder -(CH₂)₃O-;
- Z⁵ und Z⁶: -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, oder -NHCH₂(Si[(CH₃)₂]O)ₛSi[(CH₃)₂]CH₂NH-;
- Y: Wasserstoff oder Fluor;
- m, n, q und r: unabhängig voneinander 0, 1 oder 2;
- s: eine ganze Zahl von 1 bis 16;
- R¹ und R²: vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 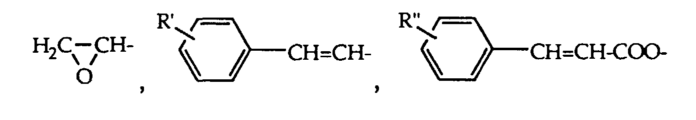
- Ph: Phenyl;
- R': niederes Alkyl;
- R'': Methyl, Methoxy, Cyano oder Halogen bedeuten, mit der Massgabe, dass R¹-Z⁵ und R²-Z⁶ keine -O-O- oder -N-O- Gruppen enthalten;
- R³: Wasserstoff, Halogen, Cyano, oder eine gegebenenfalls mit Methoxy, Cyano und/oder Halogen substituierte Alkyl-, Alkoxy-, Alkoxycarbonyl- oder Alkanoyloxy-Gruppe; und
- X¹, X² und X³: unabhängig Wasserstoff, Halogen, Cyano oder Niederalkyl bedeuten,
wobei für m=2 und/oder n=2 die beiden Ringe B und D gleich oder verschieden sein können.

Da die erfindungsgemässen Verbindungen der Formel I oder deren Mischungen eine Mesophase aufweisen, können sie auch vor dem Vernetzen auf einer Orientierungsschicht und/oder durch Anlegen eines elektrischen oder magnetischen Feldes orientiert werden. Dabei wird eine einheitliche Schicht erzeugt.

Vorzugsweise bedeutet die vernetzbare Gruppe R¹ CH₂=CH-COO-, CH₂=C(CH₃)-COO-, CH₂=C(Cl)-COO-, CH₂=C(Ph)-COO-, CH₂=CH-COO-Ph-CH₂=CH-CONH-, CH₂=C(CH₃)-CONH-, CH₂=C(Ph)-CONH-, CH₂=CH-O-, CH₂=CH-OOC-, -cis, trans -HCOO-CR'=CR'-COO-, 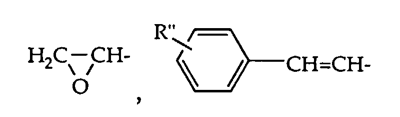 worin R' und R'' die oben angegebene Bedeutung haben.

Es sind dies Reste, welche nach Orientieren der Verbindungen der Formel I in einem Feld photochemisch vernetzt werden können.

Besonders bevorzugte Gruppen R¹ sind CH₂=CH-COO-, CH₂=C(CH₃)-COO-, CH₂=CH-O-,

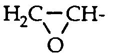

Besonders bevorzugte Gruppen X¹, X² und X³ sind Wasserstoff, Fluor, Chlor, Cyano und Methyl.

Die oben verwendeten Ausdrücke werden im folgenden erläutert:

"Gegebenenfalls mit Halogen, Methyl und/oder Cyano substituiertes 1,4-Phenylen" umfasst in der vorliegenden Erfindung 1,4-Phenylen, mit Fluor, Brom, Chlor, Methyl oder Cyano ein-oder mehrfach substituiertes 1,4-Phenylen, wie beispielsweise 2- bzw. 3-Flour-1,4-phenylen, 2,3-Difluor-1,4-phenylen, 2,6- bzw. 3,5-Difluor-1,4-phenylen, 2- bzw. 3-Chlor-1,4-phenylen, 2,3-Dichlor-1,4-phenylen, 2,6- bzw. 3,5-Dichlor-1,4-phenylen, 2-bzw. 3-Brom-1,4-phenylen, 2- bzw. 3-Methyl-1,4-phenylen, 2- bzw. 3-Cyano-1,4-phenylen und dergleichen.

"Halogen" bedeutet im Rahmen der vorliegenden Erfindung Fluor, Chlor oder Brom, insbesondere Fluor.

"Gegebenenfalls mit Methoxy, Cyano und/oder Halogen substituierte Alkyl-, Alkoxy- , Alkoxycarbonyl-, oder Alkanoyloxy-Gruppe" umfasst im Rahmen der vorliegenden Erfindung Gruppen, worin der Alkylrest geradkettig oder verzweigt sein kann und vorzugsweise 1 bis 12 Kohlenstoffatome hat. Die Gruppen können mit Methoxy, Cyano und/oder Fluor, Chlor oder Brom ein- oder mehrfach substituiert sein. Ganz bevorzugte Gruppen sind Methyl, Ethyl, Propyl, Butyl, Pentyl, Methoxy, Ethoxy, Propyloxy, Butoxy, Acetyloxy, Propanoyloxy, Butanoyloxy, Methoxycarbonyl, Ethoxycarbonyl, Propoxycarbonyl, Trifluormethyl, Trifluormethoxy, 2,2,2-Trifluorethoxy, Trifluoracetyloxy, Chlordifluormethoxy, 2-Cyanoethyl, 2-Fluorethyl, 2-Chlorethyl, 2-Bromethyl, 2-Methoxyethoxy, 2,2-Difluorethyl, 2,2,2-Trifluorethyl und dergleichen.

"Niederalkyl" umfasst im Rahmen der vorliegenden Erfindung geradkettige oder verzweigte Reste mit 1 bis 5 Kohlenstoffatomen, wie Methyl Ethyl, Propyl, Butyl, Pentyl, i-Propyl, i-Butyl, t.-Butyl und dergleichen.

Der Mesophasen-Typ der erfindungsgemässen Verbindungen kann durch Variation der Ringe in den Seiterketten beeinflusst werden. So haben aromatische Ringe wie Phenylen die Tendenz smektische Phasen zu erzeugen, während gesättigte Ringe wie trans-1,4-Cyclohexylen oder trans-1,3-Dioxan-2,5-diyl Ringe nematische Tendenzen fördern.

Bevorzugte Verbindungen haben die allgemeine Formel I-A 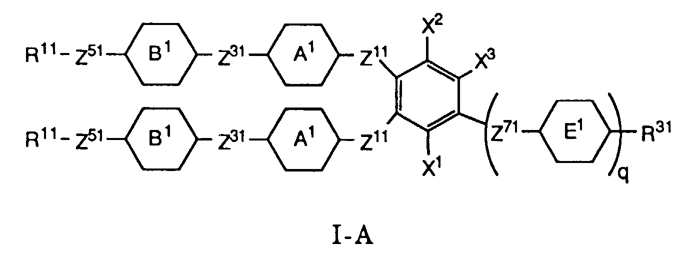 worin
- A¹, B¹ und E¹: unabhängig voneinander gegebenenfalls mit Fluor substituiertes 1,4-Phenylen, Pyridin-2,5-diyl, Pyrimidin-2,5-diyl oder trans-1,4-Cyclohexylen;
- Z¹¹: -CH₂CH₂-, -CH₂O-, -COO-, -OOC-, -(CH₂)₄- oder -(CH₂)₃O-;
- Z³¹: eine Einfachbindung, -CH₂O-, -COO- oder -OOC-;
- Z⁵¹: -(CH₂)_{s'}-, -(CH₂)_{s'}O-, -(CH₂)_{s'}COO- oder -(CH₂)_{s'}OOC-;
- Z⁷¹: -CH₂O- oder -COO-;
- s': eine ganze Zahl von 3 bis 12;
- R¹¹: CH₂=CH-COO-, CH₂=C(CH₃)-COO-, CH₂=CH-O-, 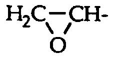
- R³¹: Halogen, Cyano, niederes Alkyl oder Alkoxycarbonyl
- q: 0 oder 1 bedeuten und
- X¹, X² und X³: die unter Formel I angegebene Bedeutung haben.

Ganz besonders bevorzugt sind Verbindungen der Formel I-A, worin q = 0 ist, wie beispielsweise Verbindungen der allgemeinen Formel Ia-Ic 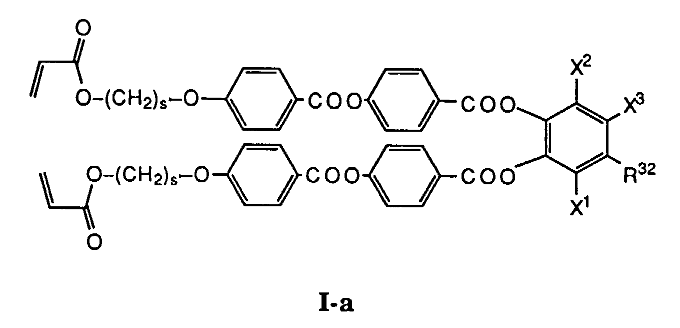 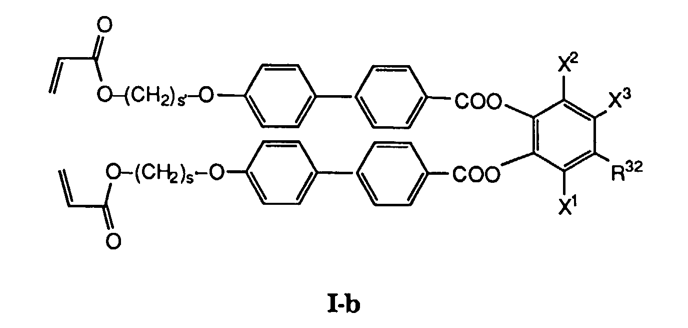 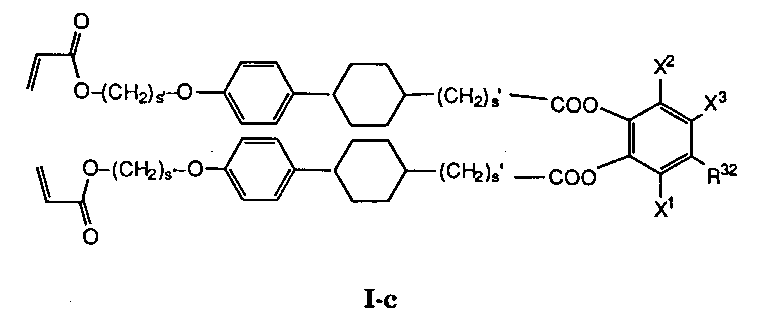 worin R³² Flour, Chlor, Cyano oder Alkoxycarbonyl bedeutet; und die übrigen Symbole wie oben definiert sind.

Ganz besonders bevorzugt sind Verbindungen der Formel I-a, worin R³² Cyano oder Alkoxycarbonyl und X¹, X² und X³ Wasserstoff bedeuten.

Ebenso bevorzugt sind Verbindungen der Formel I-B 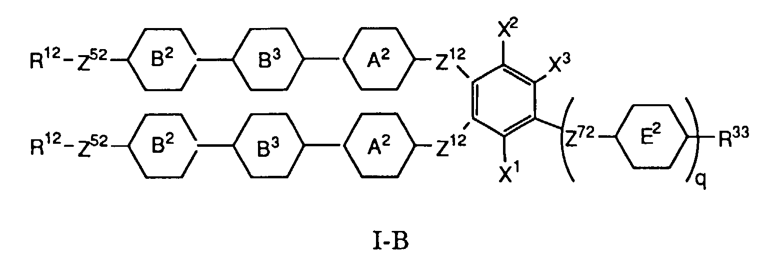 worin
- A², B², B³und E²: unabhängig voneinander gegebenenfalls mit Fluor substituiertes 1,4-Phenylen oder trans-1,4-Cyclohexylen;
- Z¹²: (CH₂)₂COO-, (CH₂)_{s'}COO- oder -(CH₂)_{s'}O-;
- Z⁵²: -(CH₂)_{s'}-, -(CH₂)_{s'}O-, -(CH₂)_{s'}COO- oder -(CH₂)_{s'}OOC-;
- Z⁷²: -CH₂O- oder -COO-;
- s': eine ganze Zahl von 3 bis 12;
- R¹²: CH₂=CH-COO-, CH₂=C(CH₃)-COO-, CH₂=CH-O-, 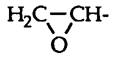
- R³³: Halogen, Cyano oder Alkoxycarbonyl bedeuten.
- q: 0 oder 1 bedeuten und
- X¹, X² und X³: die unter Formel I angegebene Bedeutung haben.

Ganz besonders bevorzugt sind Verbindungen der Formel I-B, worin q = 0 ist, wie beispielsweise Verbindungen der allgemeinen Formel Id-g 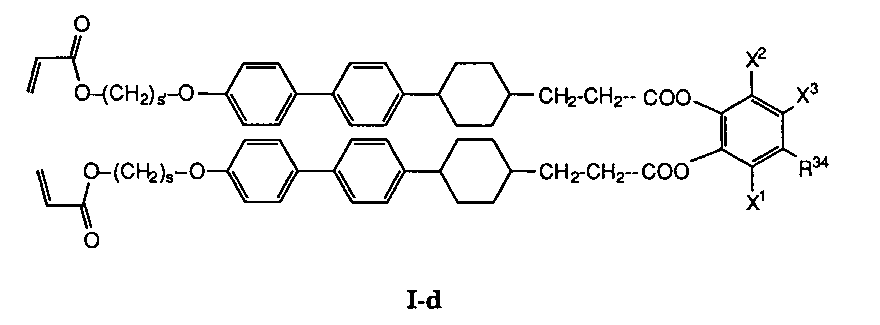 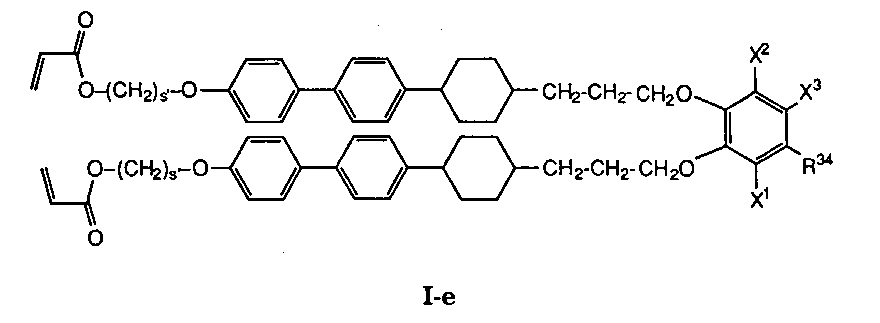 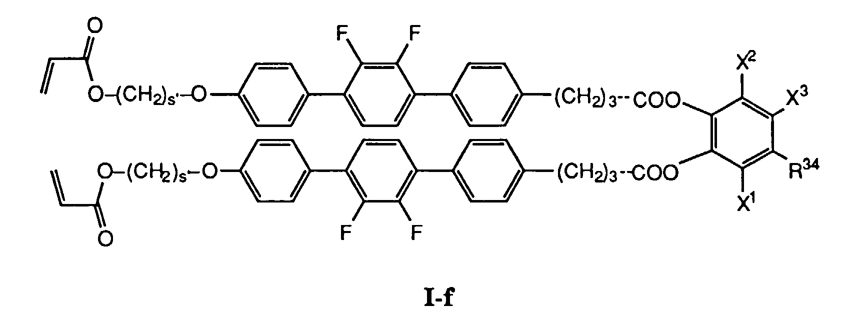 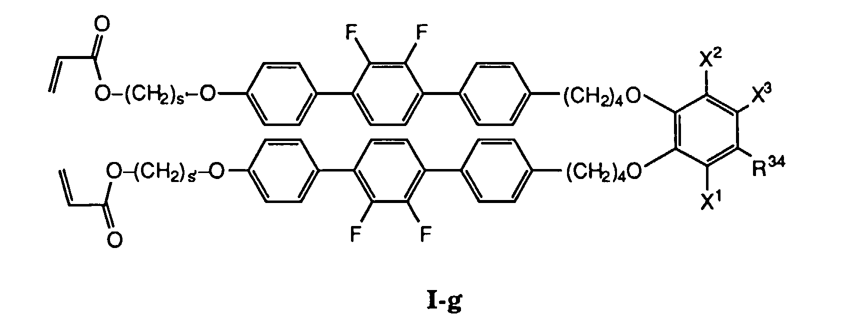 worin R³⁴ Fluor, Chlor, Cyano oder Alkoxycarbonyl bedeutet; und die übrigen Symbole wie oben definiert sind.

Die Verbindungen der Formel I-A und I-B sind synthetisch sehr einfach zugänglich und können beispielsweise analog zu den in den Schemata 1 bis 10 aufgezeigten Methoden hergestellt werden. So können in an sich bekannter Weise 2-Hydroxyphenole mit (ω-acryloyloxyalkyloxy)-substituierten Carbonsäuren umgesetzt werden. Diese Veresterung kann beispielsweise über den entsprechenden Methansulfonsäureester in Tetrahydrofuran oder in Gegenwart von N,N'-Dicyclohexylcarbodiimid (DCC) und 4-(Dimethylamino)pyridin (DMAP) in Dichlormethan oder einem anderen geeigneten Lösungsmittel, wie z.B. Chloroform, erfolgen. Die 2-Hydroxyphenole können auch in einer Mitsunobu Reaktion mit (ω-acryloyloxyalkyloxy)-substituierten Benzylalkoholen umgesetzt werden. Diese Veretherung kann beispielsweise bei Raumtemperatur in Gegenwart von Azodicarbonsäure-diethylester und Triphenylphosphin in Tetrahydrofuran oder einem anderen geeigneten Lösungsmittel, wie z.B. N, N'-Dimethylfomamid, erfolgen. Die 2-Hydroxyphenole sind bekannt oder können nach dem Fachmann bekannten Methoden hergestellt werden.

In den Schemata haben die Symbole die obengenannten Bedeutungen.

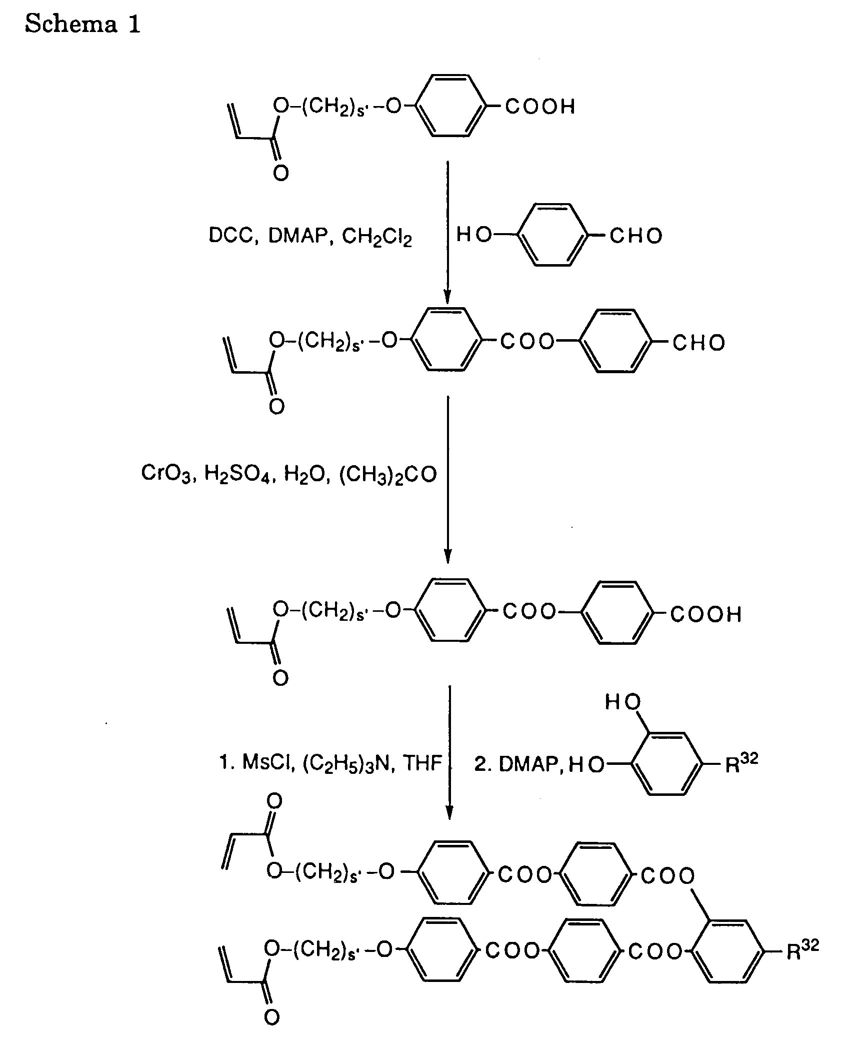

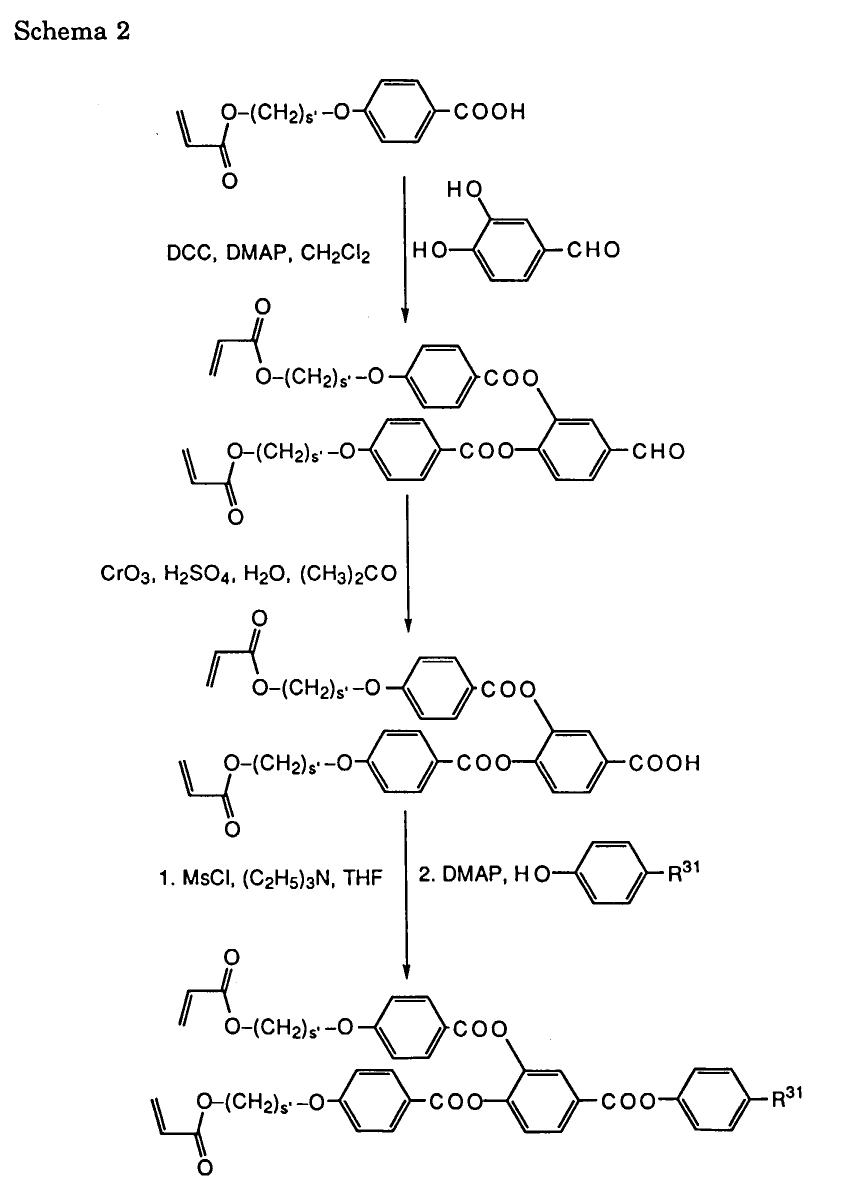

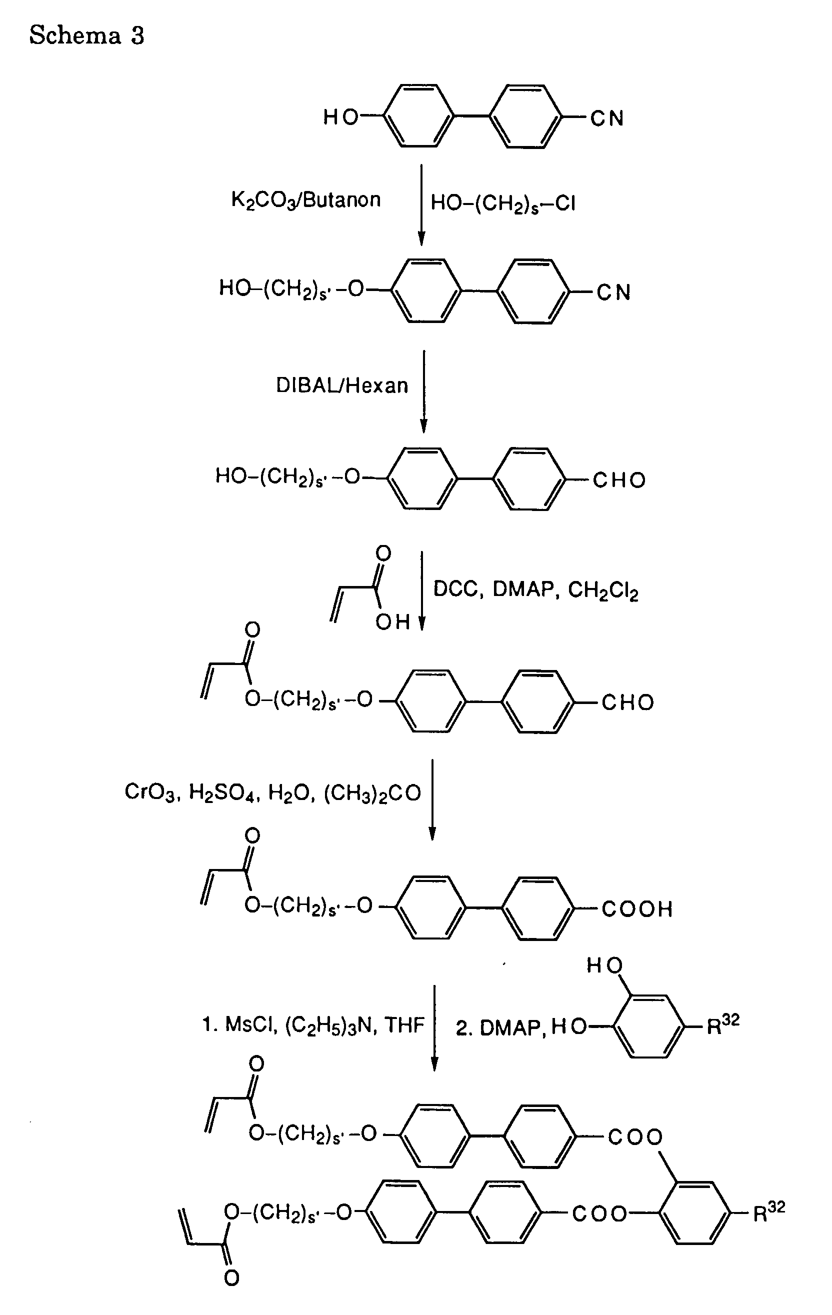

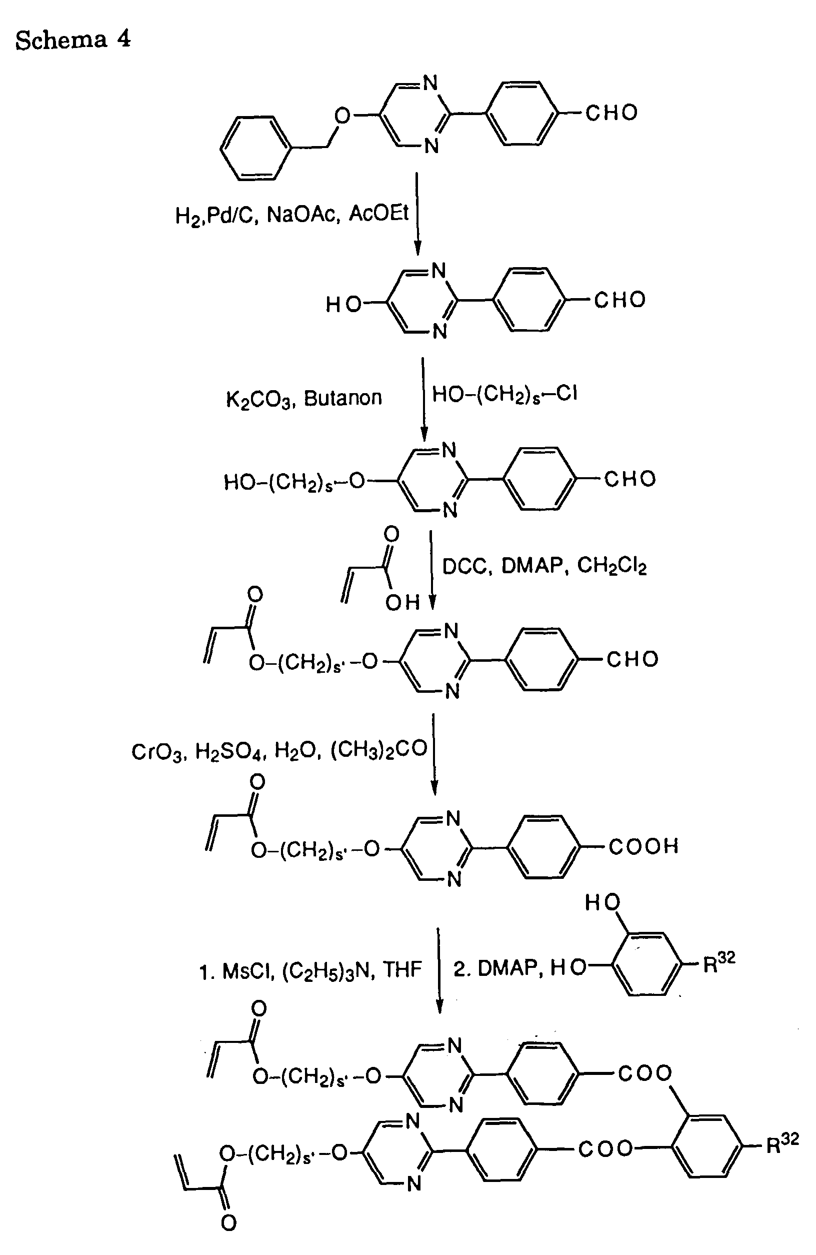

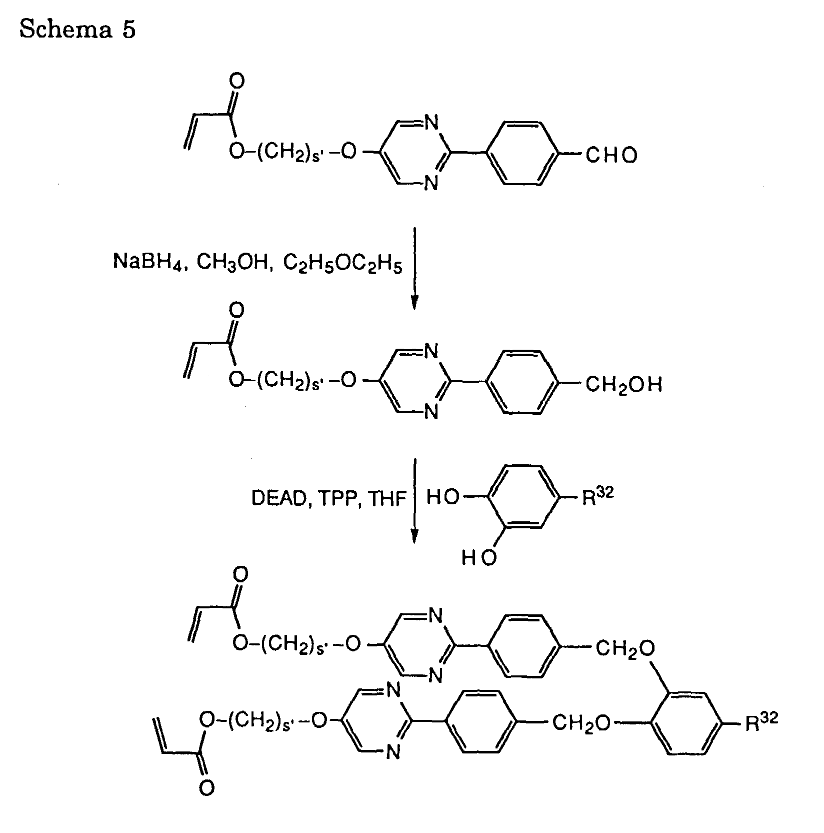

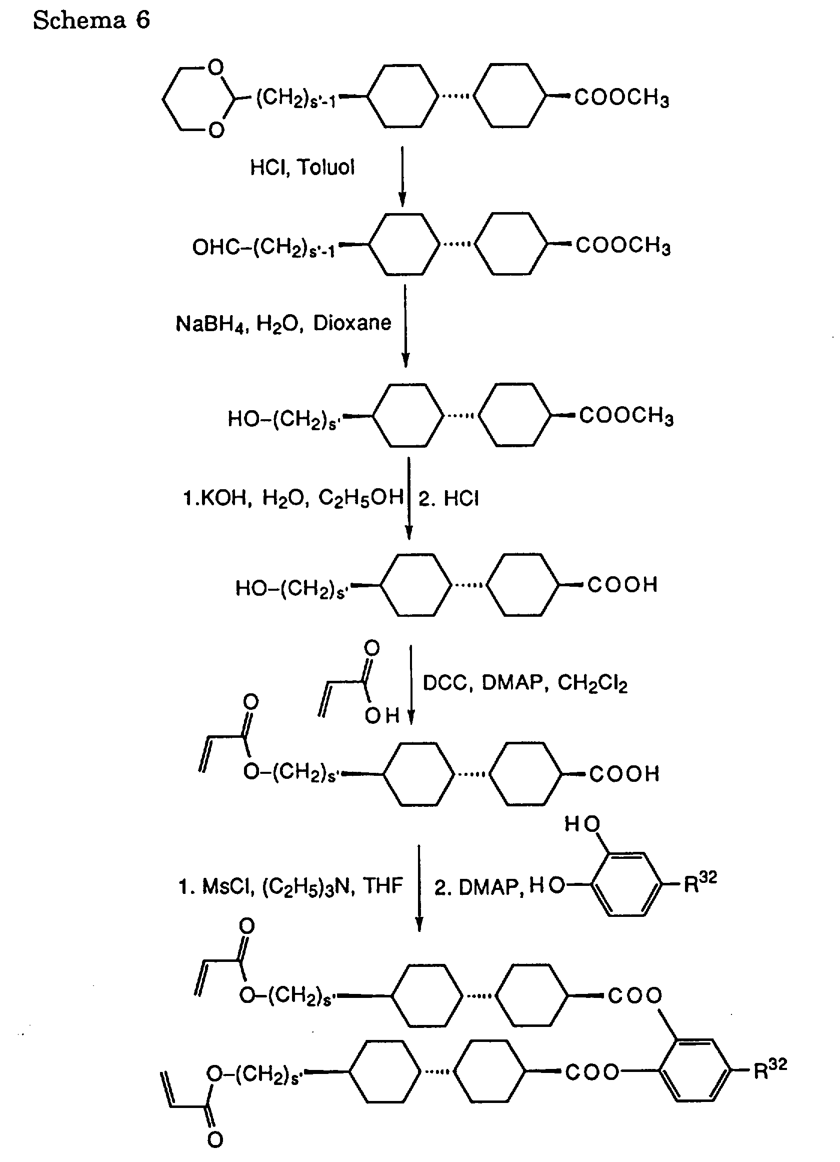

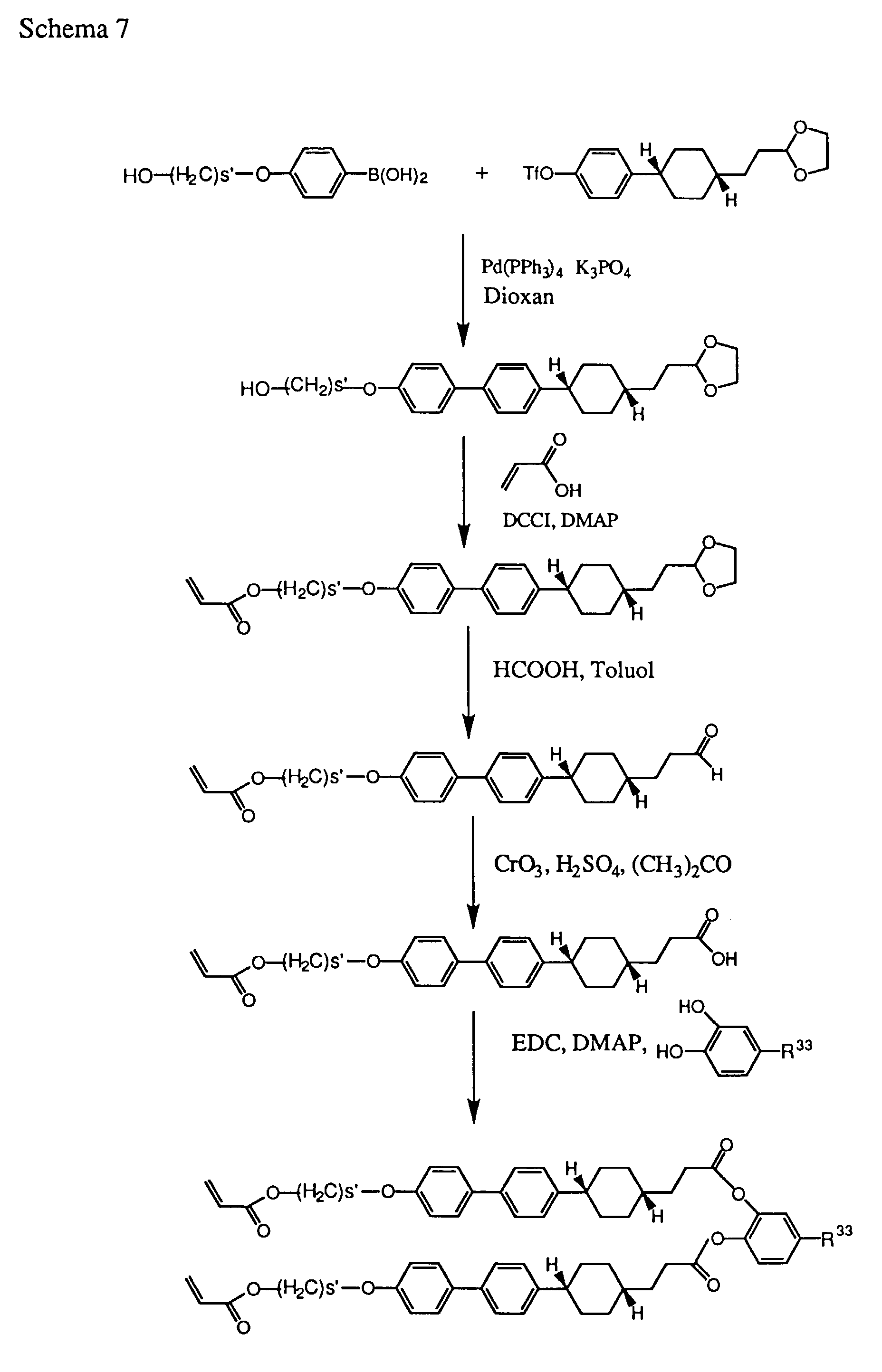

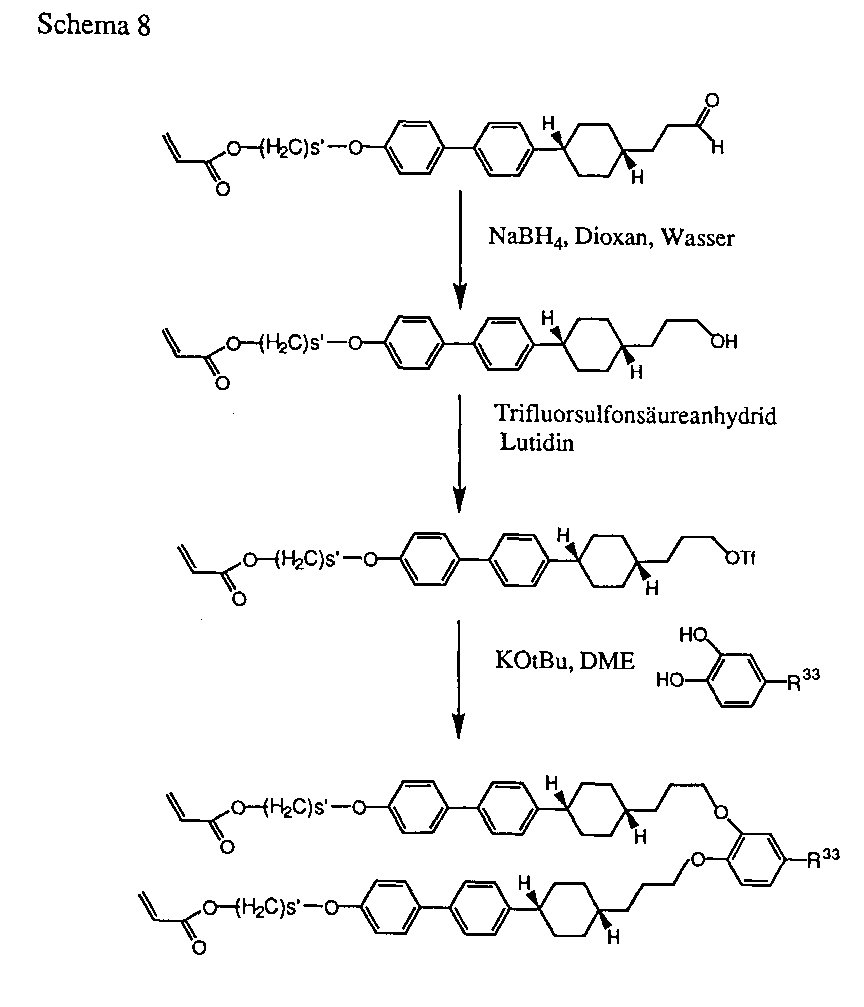

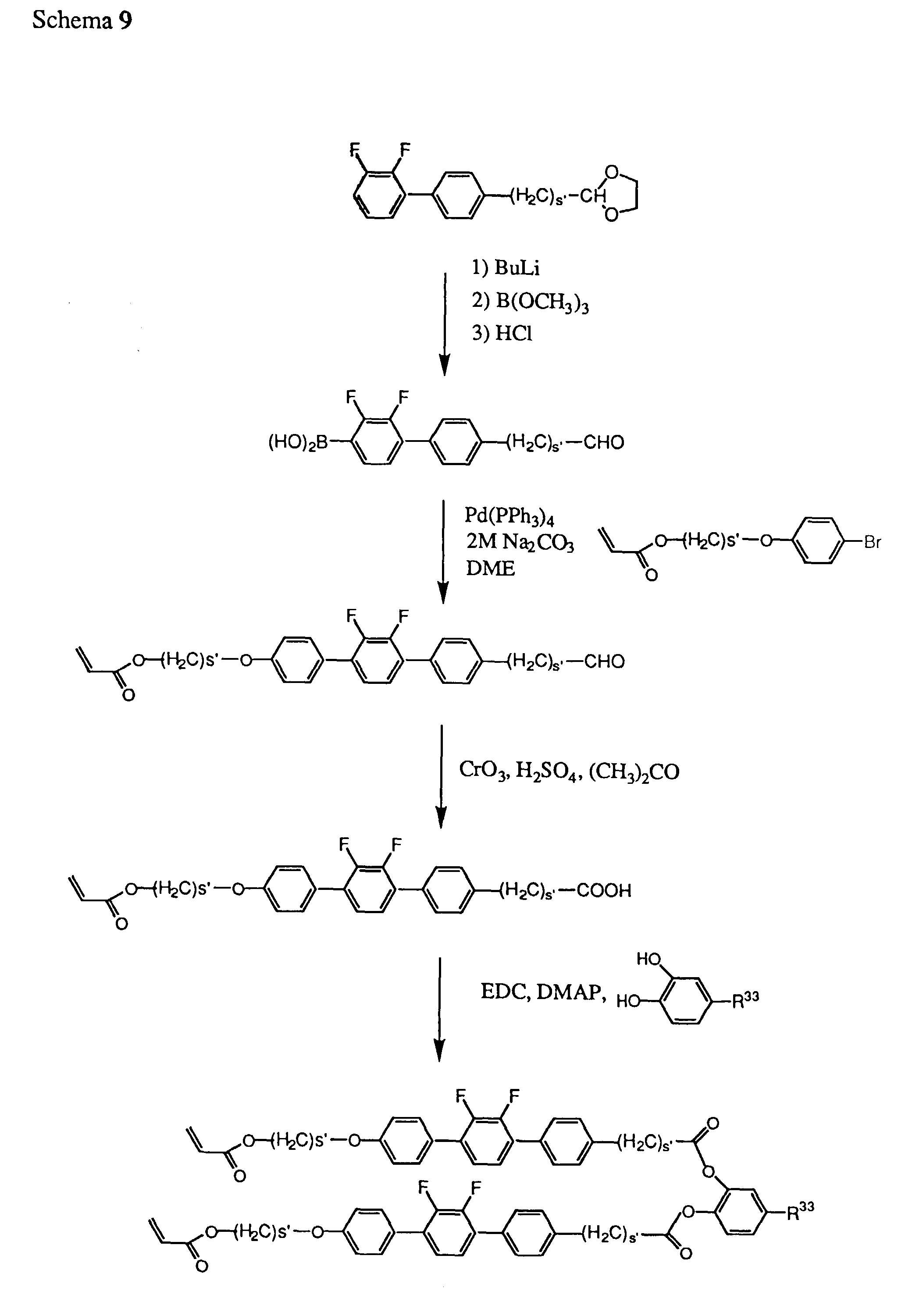

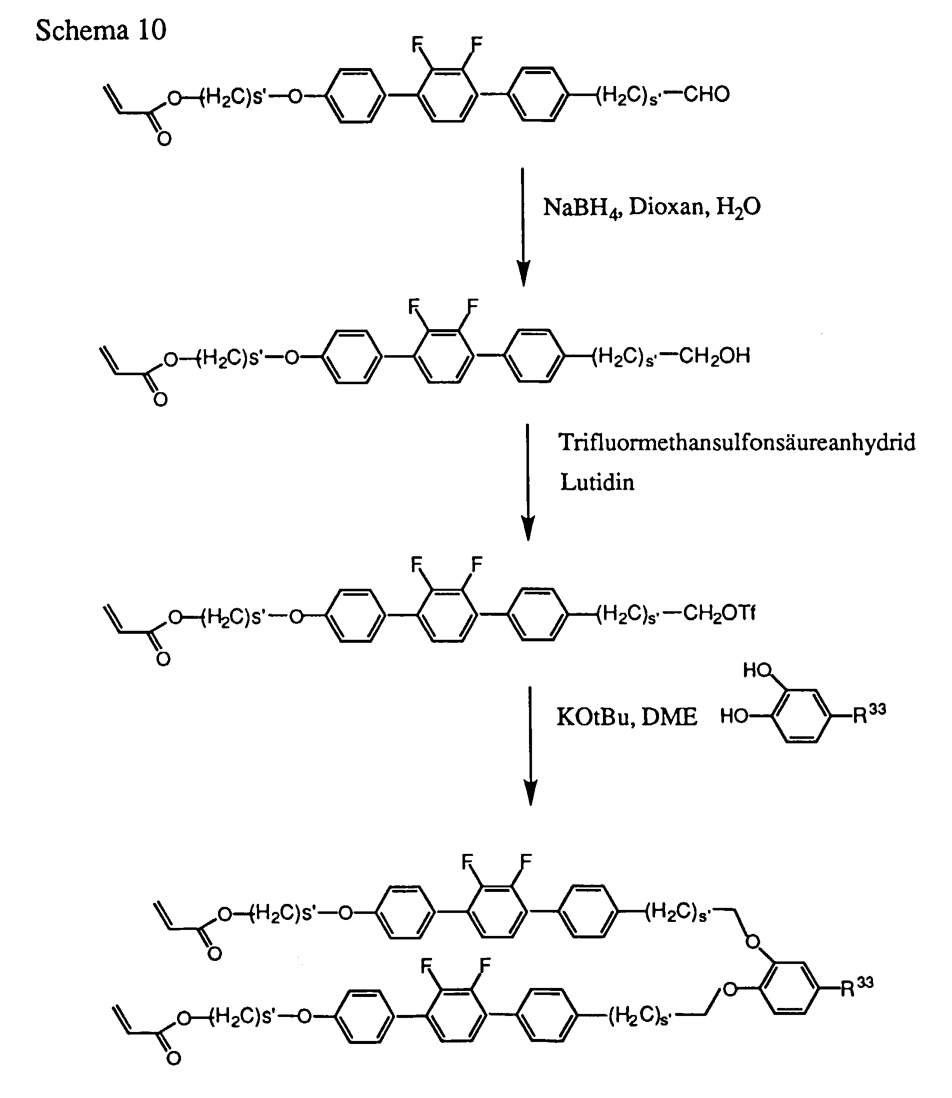

Eine kleine Menge BHT (2,6-Di-tert.-butyl-4-methyl-phenol/"Butyl-hydroxytoluol") wird jeder Stufe beigemischt, um unerwünschtes thermisches Vernetzen zu unterbinden.

Verbindungen der Formel I, worin die vernetzbaren Seitenketten verschieden sind, können durch Monoveresterung von 2-Hydroxyphenolen mit einer (ω-acryloyloxyaIkyloxy)-substituierten Carbonsäure hergestellt werden. Anschliessende Veresterung mit einer anderen (ω-acryloyloxyalkyloxy)-substituierten Carbonsäure ergibt den asymmetrischen Diester. Die entsprechenden asymmetrischen Diether sind über ein ähnliches zweistufiges Verfahren zugänglich. Die Ausgangsmaterialien sind bekannt und z.T. im Handel erhältlich.

Die Verbindungen der Formeln I können als reine Verbindungen, oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder andere, bekannte flüssigkristalline Verbindungen mit einer photovernetzbaren Gruppe sein. Es können auch eine oder mehrere chirale Komponenten im Gemisch enthalten sein.

Aufgrund der guten Löslichkeit der Verbindungen der Formel I und aufgrund ihrer guten Mischbarkeit untereinander kann der Anteil an Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 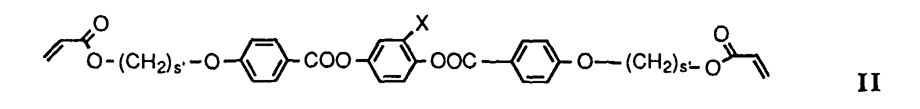 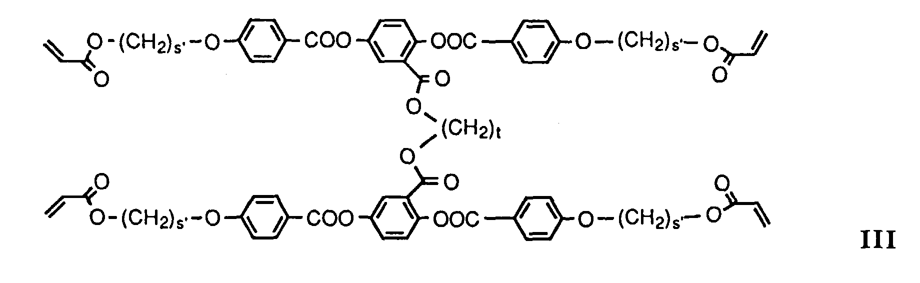 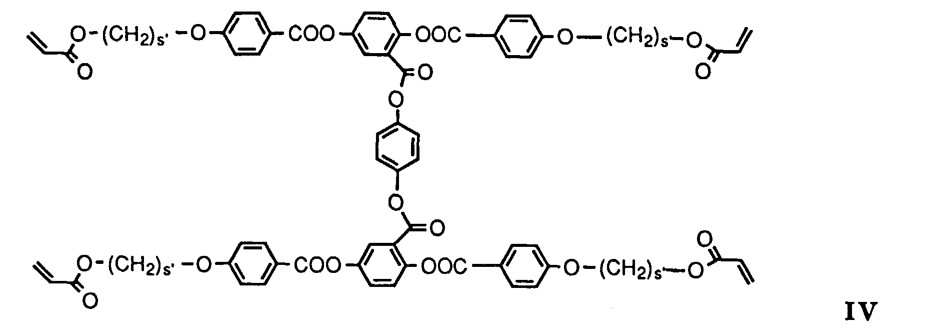 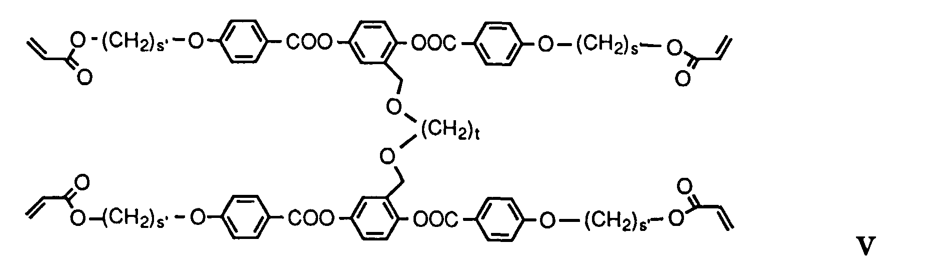 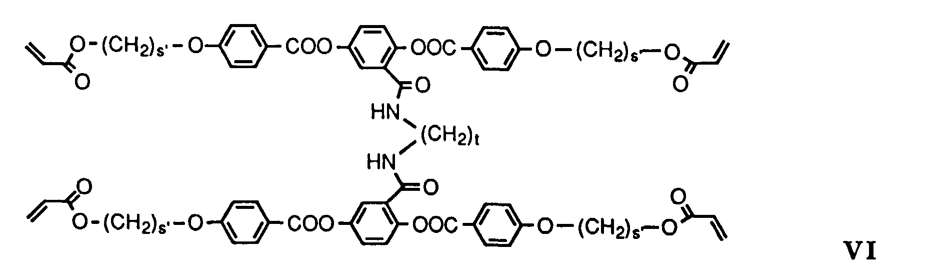 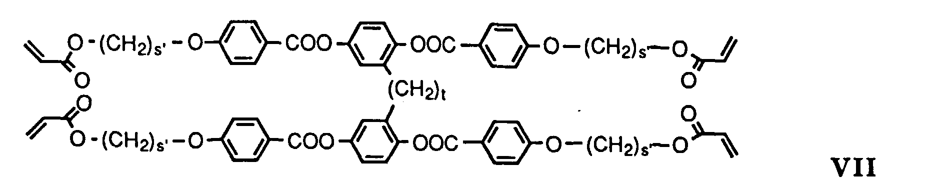 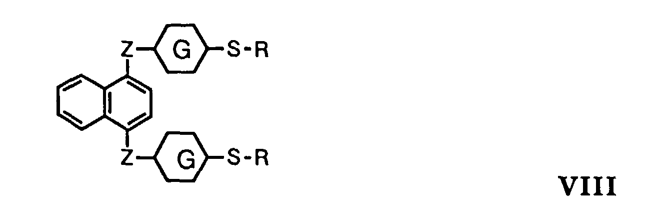 und 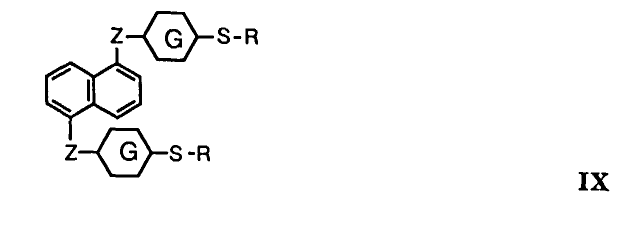 worin
- X: Wasserstoff, Fluor, Chlor, Brom oder Methyl;
- s': eine ganze Zahl von 3 bis 12; und
- t: eine ganze Zahl von 2 bis 12 ist;
- Z: -OCH₂- oder -OOC-;
- G: 1,4-Phenylen oder 2- bzw. 3-Fluor-1,4-phenylen;
- S: -(CH₂)_{s'} - oder -(CH₂)_{s'}O-; und
- R: CH₂=CH-COO-, CH₂=C(CH₃)-COO-, CH₂=CH-O-, 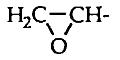 bedeuten.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase.

### Beispiel 1

Zu einer Lösung von 2,0 g 4-(4-[8-Acryloyloxyoctyloxy]phenylcarbonyloxy)benzoesäure in 40 ml Tetrahydrofuran wurde unter Rühren bei -25^{o}C 0,9 g Triethylamin und dann 0,5 g Methansulfochlorid zugetropft. Das Reaktionsgemisch wurde 1 Stunde bei -25^{o}C gerührt, mit 0,28 g 3,4-Dihydroxybenzonitril und 0,05 g 4-(Dimethylamino)pyridin versetzt, über Nacht bei Raumtemperatur gerührt, auf 100 ml Wasser gegossen und dann dreimal mit je 50 ml Diethylether extrahiert. Die organischen Phasen wurden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergab 1,2 g 3,4-bis[4-(4-[8-Acryloyloxyoctyloxy]phenylcarbonyloxy)phenylcarbonyloxy]benzonitril; Smp. (C-S_{A}) 70^{o}C, S_{A}-N 139^{o}C, Klp. (N-I) 152^{o}C.

Die als Ausgangsmaterial verwendete 4-(4-[8-Acryloyloxyoctyloxy]phenylcarbonyloxy)benzoesäure wurde wie folgt hergestellt:
(a). Zu einer Lösung von 1,9 g 4-Hydroxybenzaldehyd, 5,0 g 4-[8-Acryloyloxyoctyloxy]benzoesäure und 0,1 g 4-(Dimethylamino)pyridin in 100 ml Dichlormethan wurde unter Rühren bei 0°C innert 15 Minuten 3,9 g N,N'-Dicyclohexylcarbodiimid gegeben. Das Reaktionsgemisch wurde über Nacht bei Raumtemperatur gerührt, filtriert, und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus Ethylacetat ergab 5,5 g 4-(4-[8-Acryloyloxyoctyloxy]phenylcarbonyloxy)benzaldehyd; Smp. 73^{o}C.
(b). Eine Lösung von 5,5 g 4-(4-[8-Acryloyloxyoctyloxy]phenylcarbonyloxy)benzaldehyd in 100 ml Aceton wurde tropfenweise mit 25 ml Jones' Reagenz versetzt. Das Gemisch wurde über Nacht bei Raumtemperatur gerührt, auf 100 ml Wasser gegossen, dreimal mit je 50 ml Ethylacetat extrahiert. Die organischen Phasen wurden vereinigt, zweimal mit je 100 ml Wasser gewaschen, über Magnesiumsulfat getrocknet, filtriert und das Filtrat eingeengt. Der Rückstand wurde aus Ethylacetat umkristallisiert. Dies ergab 3,5 g 4-(4-[8-Acryloyloxyoctyloxy]phenylcarbonyloxy)benzoesäure; Smp. (C-N) 116^{o}C, Klp. (N-I) 240^{o}C (Zersetzung).

In analoger Weise können folgende Verbindungen hergestellt werden:
3,4-bis[4-(4-[3-Acryloyloxypropyloxy]phenylcarbonyloxy)phenylcarbonyloxy]benzonitril;
3,4-bis[4-(4-[4-Acryloyloxybutyloxy]phenylcarbonyloxy)phenylcarbonyloxy]benzonitril;
3,4-bis[4-(4-[5-Acryloyloxypentyloxy]phenylcarbonyloxy)phenylcarbonyloxy]benzonitril;
3,4-bis[4-(4-[6-Acryloyloxyhexyloxy]phenylcarbonyloxy)phenylcarbonyloxy]benzonitril;
3,4-bis[4-(4-[7-Acryloyloxyheptyloxy]phenylcarbonyloxy)phenylcarbonyloxy]benzonitril;
3,4-bis[4-(4-[9-Acryloyloxynonyloxy]phenylcarbonyloxy)phenylcarbonyloxy]benzonitril;
3,4-bis[4-(4-[10-Acryloyloxydecyloxy]phenylcarbonyloxy)phenylcarbonyloxy]benzonitril;
3,4-bis[4-(4-[11-Acryloyloxyundecyloxy]phenylcarbonyloxy)phenylcarbonyloxy]benzonitril;
3,4-bis[4-(4-[12-Acryloyloxydodecyloxy]phenylcarbonyloxy)phenylcarbonyloxy]benzonitril;
1,2-bis[4-(4-[8-Acryloyloxyoctyloxy]phenylcarbonyloxy)phenylcarbonyloxy]benzol; Smp. (C- S_{A}) 89°C, S_{A}-N, 105°C, Klp. (N-I) 107°C;
Methyl 3,4-bis[4-(4-[8-Acryloyloxyoctyloxy]phenylcarbonyloxy)phenylcarbonyloxy]benzoesäureester;
Ethyl 3,4-bis[4-(4-[8-Acryloyloxyoctyloxy]phenylcarbonyloxy)phenylcarbonyloxy]benzoesäureester; Smp. (C- S_{A}) 25°C, Klp. (S_{A}-I) 150°C;
3,4-bis[4-(4-[8-Acryloyloxyoctyloxy]phenylcarbonyloxy)phenylcarbonyloxy]toluol;
3,4-bis[4-(4-[8-Acryloyloxyoctyloxy]phenylcarbonyloxy)phenylcarbonyloxy]-1-ethylbenzol;
3,4-bis[4-(4-[8-Acryloyloxyoctyloxy]phenylcarbonyloxy)phenylcarbonyloxy]-1-propylbenzol.

### Beispiel 2

Zu einer Lösung von 0,4 g 3,4-bis(4-[8-Acryloyloxyoctyloxy]phenylcarbonyloxy)benzoesäure in 40 ml Tetrahydrofuran wurde unter Rühren bei -25^{o}C 0,9 g Triethylamin und dann 0,5 g Methansulfochlorid zugetropft. Das Reaktionsgemisch wurde 1 Stunde bei -25^{o}C gerührt, dann mit 0,06 g 2-Fluor-4-hydroxybenzonitril und 0,05 g 4-(Dimethylamino)pyridin in 40 ml Tetrahydrofuran versetzt, über Nacht bei Raumtemperatur gerührt, auf 100 ml Wasser gegossen und dann dreimal mit je 50 ml Diethylether extrahiert. Die organischen Phasen wurden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus Ethylalkohol ergab 1,2 g 3,4-bis[4-(4-[8-Acryloyloxyoctyloxy]-phenylcarbonyloxy)phenylcarbonyloxy]-2-fluorbenzonitril; Smp. (C-I) 85^{o}C, Klp. (N-I) 38^{o}C.

Die als Ausgangsmaterial verwendete 3,4-bis(4-[8-Acryloyloxyoctyloxy]phenylcarbonyloxy)benzoesäure wurde wie folgt hergestellt:
(a). Zu einer Lösung von 1,0 g 4-(8-Acryloyloxyoctyloxy)benzoesäure in 40 ml Tetrahydrofuran wurde unter Rühren bei -25^{o}C 0,6 g Triethylamin und dann 0,4 g Methansulfochlorid zugetropft. Das Reaktionsgemisch wurde 1 Stunde bei -25^{o}C gerührt, dann mit 0,20 g 4-Hydroxybenzaldehyd und 0,03 g 4-(Dimethylamino)pyridin in 40 ml Tetrahydrofuran versetzt, über Nacht bei Raumtemperatur gerührt, auf 100 ml Wasser gegossen und dann dreimal mit je 50 ml Diethylether extrahiert. Die organischen Phasen wurden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergab 0,6 g 3,4-bis(4-[8-Acryloyloxyoctyloxy]phenylcarbonyloxy)benzaldehyd; Smp. (C-I) 56^{o}C.
(b). Eine Lösung von 0,6 g 3,4-bis(4-[8-Acryloyloxyoctyloxy]phenylcarbonyloxy)benzaldehyd in 20 ml Aceton wurde bei 0°C tropfenweise mit 2 ml Jones' Reagenz versetzt. Das Gemisch wurde 1 Stunde bei 0°C und dann über Nacht bei Raumtemperatur gerührt, auf 100 ml Wasser gegossen und filtriert, das Filtergut mit Wasser gewaschen und dann getrocknet. Dies ergab 0,4 g 3,4-bis(4-[8-Acryloyloxyoctyloxy]phenylcarbonyloxy)benzoesäure.

In analoger Weise können folgende Verbindungen hergestellt werden:
3,4-bis[4-(4-[3-Acryloyloxypropyloxy]phenylcarbonyloxy)phenylcarbonyloxy]-2-fluorbenzonitril;
3,4-bis[4-(4-[4-Acryloyloxybutyloxy]phenylcarbonyloxy)phenylcarbonyloxy]-2-fluorbenzonitril;
3,4-bis[4-(4-[5-Acryloyloxypentyloxy]phenylcarbonyloxy)phenylcarbonyloxy]-2-fluorbenzonitril;
3,4-bis[4-(4-[6-Acryloyloxyhexyloxy]phenylcarbonyloxy)phenylcarbonyloxy]-2-fluorbenzonitril;
3,4-bis[4-(4-[7-Acryloyloxyheptyloxy]phenylcarbonyloxy)phenylcarbonyloxy]-2-fluorbenzonitril;
3,4-bis[4-(4-[9-Acryloyloxynonyloxy]phenylcarbonyloxy)phenylcarbonyloxy]-2-fluorbenzonitril;
3,4-bis[4-(4-[10-Acryloyloxydecyloxy]phenylcarbonyloxy)phenylcarbonyloxy]-2-fluorbenzonitril;
3,4-bis[4-(4-[11-Acryloyloxyundecyloxy]phenylcarbonyloxy)phenylcarbonyloxy]-2-fluorbenzonitril;
3,4-bis[4-(4-[12-Acryloyloxydodecyloxy]phenylcarbonyloxy)phenylcarbonyloxy]-2-fluorbenzonitril;
3,4-bis[4-(4-[6-Acryloyloxyhexyloxy]phenylcarbonyloxy)phenylcarbonyloxy]-1-fluorbenzol;
3,4-bis[4-(4-[6-Acryloyloxyhexyloxy]phenylcarbonyloxy)phenylcarbonyloxyl-1-chlorbenzol;
3,4-bis[4-(4-[6-Acryloyloxyhexyloxy]phenylcarbonyloxy)phenylcarbonyloxy]-1-brombenzol;;
3,4-bis[4-(4-[6-Acryloyloxyhexyloxy]phenylcarbonyloxy)phenylcarbonyloxy]-1, 2-difluorbenzol;
3,4-bis[4-(4-[6-Acryloyloxyhexyloxy]phenylcarbonyloxy)phenylcarbonyloxy]-1, 2-dichlorbenzol;
3,4-bis[4-(4-[6-Acryloyloxyhexyloxy]phenylcarbonyloxy)phenylcarbonyloxy]-1, 2-dicyanobenzol;
3,4-bis[4-(4-[6-Acryloyloxyhexyloxy]phenylcarbonyloxy)phenylcarbonyloxy]-1-chlor- 2-fluorbenzol.

### Beispiel 3

Zu einer Lösung von 2,0 g 4-(8-Acryloyloxyoctyloxy)biphenyl-4'-carbonsäure in 40 ml Tetrahydrofuran wird unter Rühren bei -25^{o}C 0,9 g Triethylamin und dann 0,5 g Methansulfochlorid zugetropft. Das Reaktionsgemisch wird 1 Stunde bei -25^{o}C gerührt, dann mit 0,25 g 3,4-Dihydroxy-benzonitril und 0,05 g 4-(Dimethylamino)pyridin versetzt, über Nacht bei Raumtemperatur gerührt, auf 100 ml Wasser gegossen und dann dreimal mit je 50 ml Diethylether extrahiert. Die organischen Phasen werden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,0 g 3,4-bis[4-(8-Acryloyloxyoctyloxy)biphenyl-4'-carbonyloxy]benzonitril.

Die als Ausgangsmaterial verwendete 4-(8-Acryloyloxyoctyloxy)biphenyl-4'-carbonsäure wird wie folgt hergestellt:
(a). Eine Lösung von 5,0 g 4'-Hydroxybiphenyl-4-carbonsäurenitril und 5,1 g 8-Chlor-1-octanol in 100 ml Ethylmethylketon wird mit 14,1 g fein pulverisiertem Kaliumcarbonat versetzt und das Gemisch über Nacht unter leichtem Rückfluss erwärmt. Die Suspension wird genutscht und das Filtrat im Vakuum eingeengt. Chromatographie des Rückstands an Kieselgel mit Hexan/Ethylacetat (Vol. 1:1) ergibt 4,9 g 4'-(8-Hydroxyoctyloxy)biphenyl-4-carbonsäurenitril.
(b). Einer Lösung von 4,9 g 4-Cyano-4'-(8-hydroxyoctyloxy)biphenyl in 25 ml Toluol wird bei 0°C 50 ml einer 1, 2 M Lösung von Diisobutylaluminiumhydrid in Toluol versetzt und das Gemisch über Nacht bei Raumtemperatur gerührt. Das Reaktionsgemisch wird auf 100 ml 1,0 N Schwefelsäure gegossen und zweimal mit je 50 ml Ethylacetat extrahiert. Die organischen Phasen werden mit 50 ml Wasser gewaschen, über Magnesiumsulfat getrocknet und eingeengt. Chromatographie des Rückstandes an Kieselgel mit Hexan/Ethylacetat (Vol. 8:2) und Umkristallisation der gemäss Dünnschichtchromatographie reinen Fraktionen aus Ethylalkohol ergibt 3,8 g 4-Formyl-4'-(8-hydroxyoxyoctyloxy)biphenyl.
(c). Zu einer Lösung von 3,8 g 4-Formyl-4'-(8-hydroxyoctyloxy)biphenyl, 0,9 g Acrylsäure und 0,05 g 4-(Dimethylamino)pyridin in 25 ml Dichlormethan wird unter Rühren innert 5 Minuten 2,9 g N,N '-Dicyclohexylcarbodiimid gegeben. Das Reaktionsgemisch wird über Nacht weiter gerührt, filtriert und dann eingeengt. Chromatographie des Rückstandes an Kieselgel mit Hexan/Ethylacetat (Vol. 8:2) und Umkristallisation der gemäss Dünnschichtchromatographie reinen Fraktionen aus Ethylalkohol ergibt 2,5 g 4-Formyl-4'-(8-acryloyloxyoctyloxy)biphenyl.
(d). Eine Lösung von 2,5 g 4-Formyl-4'-(8-acryloyloxyoctyloxy)biphenyl in 100 ml Aceton wird tropfenweise mit 10 ml Jones' Reagenz versetzt. Das Gemisch wird 1 Stunde bei Raumtemperatur und dann über Nacht bei Raumtemperatur gerührt, auf 100 ml Wasser gegossen und dann dreimal mit je 50 ml Ethylacetat extrahiert. Die vereinigten organischen Phasen werden zweimal mit je 100 ml Wasser gewaschen, über Magnesiumsulfat getrocknet, filtriert und das Filtrat eingeengt. Der Rückstand wird aus Ethylacetat umkristallisiert. Dies ergibt 1,6 g 4-(8-Acryloyloxyoctyloxy)biphenyl-4'-carbonsäure.

In analoger Weise können folgende Verbindungen hergestellt werden:
3,4-bis[4-(3-Acryloyloxypropyloxy)biphenyl-4'-carbonyloxy]benzonitril;
3,4-bis[4-(4-Acryloyloxybutyloxy)biphenyl-4'-carbonyloxy]benzonitril;
3,4-bis[4-(5-Acryloyloxypentyloxy)biphenyl-4'-carbonyloxy]benzonitril;
3,4-bis[4-(6-Acryloyloxyhexyloxy)biphenyl-4'-carbonyloxy]benzonitril;
3,4-bis[4-(7-Acryloyloxyheptyloxy)biphenyl-4'-carbonyloxy]benzonitril;
3,4-bis[4-(9-Acryloyloxynonyloxy)biphenyl-4'-carbonyloxy]benzonitril;
3,4-bis[4-(10-Acryloyloxydecyloxy)biphenyl-4'-carbonyloxy]benzonitril;
3,4-bis[4-(11-Acryloyloxyundecyloxy)biphenyl-4'-carbonyloxy]benzonitril;
3,4-bis[4-(12-Acryloyloxydodecyloxy)biphenyl-4'-carbonyloxy]benzonitril;
Methyl 3,4-bis[4-(8-acryloyloxyoctyloxy)biphenyl-4'-carbonyloxy]benzoesäureester;
Ethyl 3,4-bis[4-(8-acryloyloxyoctyloxy)biphenyl-4'-carbonyloxy]benzoesäureester;
Propyl 3,4-bis[4-(8-acryloyloxyoctyloxy)biphenyl-4'-carbonyloxy]benzoesäureester;
3,4-bis[4-(8-Acryloyloxyoctyloxy)biphenyl-4'-carbonyloxy]toluol;
3,4-bis[4-(8-Acryloyloxyoctyloxy)biphenyl-4'-carbonyloxy]-1-ethylbenzol;
3,4-bis[4-(8-Acryloyloxyoctyloxy)biphenyl-4'-carbonyloxy]-1-propylbenzol;

### Beispiel 4

Zu einer Lösung von 2,0 g 4-(5-[8-Acryloyloxyoctyloxy]pyrimidin-2-yl)benzoesäure in 40 ml Tetrahydrofuran wird unter Rühren bei -25^{o}C 0,9 g Triethylamin und dann 0,5 g Methansulfochlorid zugetropft. Das Reaktionsgemisch wird 1 Stunde bei -25^{o}C gerührt, dann mit 0,28 g 3,4-Dihydroxybenzonitril und 0,05 g 4-(Dimethylamino)pyridin versetzt, über Nacht bei Raumtemperatur gerührt, auf 100 ml Wasser gegossen und dann dreimal mit je 50 ml Diethylether extrahiert. Die organischen Phasen werden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,1 g 3,4-bis[4-(5-[8-Acryloyloxyoctyloxy]pyrimidin-2-yl)phenylcarbonyloxy]benzonitril.

Die als Ausgangsmaterial verwendete 4-(5-[8-Acryloyloxyoctyloxy]pyrimidin-2-yl)benzoesäure wird wie folgt hergestellt:
(a). Ein Gemisch aus 10,0 g 4-(5-Benzyloxypyrimidin-2-yl)benzaldehyd, 0,2 g Natriumcarbonat und 250 ml Ethylacetat wird mit 1,0 g Palladium auf Aktivkohle (10%) versetzt, und bei Normaldruck und Raumtemperatur bis zum Stillstand der Wasserstoffaufnahme hydriert. Das anorganische Material wird über Sillit abfiltriert und das Filtrat eingeengt. Umkristallisation des Rohproducktes aus Hexan ergibt 6,1 g reinen 4-(5-Hydroxypyrimidin-2-yl)benzaldehyd.
(b). Eine Lösung von 6,1 g 4-(5-Hydroxypyrimidin-2-yl)benzaldehyd und 6,0 g 8-Chlor-1-octanol in 100 ml Ethyl-methylketon wird mit 16,9 g fein pulverisiertem Kaliumcarbonat versetzt und das Gemisch über Nacht unter leichtem Rückfluss erwärmt. Die Suspension wird genutscht und das Filtrat im Vakuum eingeengt. Chromatographie des Rückstands an Kieselgel mit Hexan/Ethylacetat (Vol. 1:1) ergibt 5,5 g 4-(5-[8-Hydroxyoctyloxy]pyrimidin-2-yl)benzaldehyd.
(c). Zu einer Lösung von 5,5 g 4-(5-[8-Hydroxyoctyloxy]pyrimidin-2-yl)benzaldehyd, 1,2 g Acrylsäure und 0,2 g 4-(Dimethylamino)pyridin in 25 ml Dichlormethan wird unter Rühren innert 5 Minuten 4,2 g N,N '-Dicyclohexylcarbodiimid gegeben. Das Reaktionsgemisch wird über Nacht gerührt, filtriert und das Filtrat eingeengt. Chromatographie des Rückstandes an Kieselgel mit Hexan/Ethylacetat (Vol. 8:2) und Umkristallisation der gemäss Dünnschichtchromatographie reinen Fraktionen aus Ethylalkohol ergibt 4,8 g 4-(5-[8-Acryloyloxyoctyloxy]pyrimidin-2-yl)benzaldehyd.
(d). Eine Lösung von 3,2 g 4-(5-[8-Acryloyloxyoctyloxy]pyrimidin-2-yl)benzaldehyd in 100 ml Aceton wird tropfenweise mit 20 ml Jones' Reagenz versetzt. Das Gemisch wird über Nacht bei Raumtemperatur gerührt, auf 100 ml Wasser gegossen und dann dreimal mit je 50 ml Ethylacetat extrahiert. Die organischen Phasen werden vereinigt, zweimal mit je 100 ml Wasser gewaschen, über Magnesiumsulfat getrocknet, filtriert und das Filtrat eingeengt. Der Rückstand wird aus Ethylacetat umkristallisiert. Dies ergibt 2,0 g4-(5-[8-Acryloyloxyoctyloxy]pyrimidin-2-yl)benzoesäure.

In analoger Weise können folgende Verbindungen hergestellt werden:
3,4-bis[4-(5-[3-Acryloyloxypropyloxy]pyrimidin-2-yl)phenylcarbonyloxy]benzonitril;
3,4-bis[4-(5-[4-Acryloyloxybutyloxy]pyrimidin-2-yl)phenylcarbonyloxy]benzonitril;
3,4-bis[4-(5-[5-Acryloyloxypentyloxy]pyrimidin-2-yl)phenylcarbonyloxy]benzonitril;
3,4-bis[4-(5-[6-Acryloyloxyhexyloxy]pyrimidin-2-yl)phenylcarbonyloxy]benzonitril;
3,4-bis[4-(5-[7-Acryloyloxyheptyloxy]pyrimidin-2-yl)phenylcarbonyloxy]benzonitril;
3,4-bis[4-(5-[9-Acryloyloxynonyloxy]pyrimidin-2-yl)phenylcarbonyloxy]benzonitril;
3,4-bis[4-(5-[10-Acryloyloxydecyloxy]pyrimidin-2-yl)phenylcarbonyloxy]benzonitril;
3,4-bis[4-(5-[11-Acryloyloxyundecyloxy]pyrimidin-2-yl)phenylcarbonyloxy]benzonitril;
3,4-bis[4-(5-[12-Acryloyloxydodecyloxy]pyrimidin-2-yl)phenylcarbonyloxy]benzonitril;
Methyl 3,4-bis[4-(5-[8-acryloyloxyoctyloxy]pyrimidin-2-yl)phenylcarbonyloxy]benzoesäureester;
Ethyl 3,4-bis[4-(5-[8-acryloyloxyoctyloxy]pyrimidin-2-yl)phenylcarbonyloxy]benzoesäureester;
Propyl 3,4-bis[4-(5-[8-acryloyloxyoctyloxy]pyrimidin-2-yl)phenylcarbonyloxy]benzoesäureester;
3,4-bis[4-(5-[12-Acryloyloxydodecyloxy]pyrimidin-2-yl)phenylcarbonyloxy]toluol;
3,4-bis[4-(5-[12-Acryloyloxydodecyloxy]pyrimidin-2-yl)phenylcarbonyloxy]-1-ethylbenzol;
3,4-bis[4-(5-[12-Acryloyloxydodecyloxy]pyrimidin-2-yl)phenylcarbonyloxy]-1-propylbenzol;
3,4-bis[2-(4-[3-Acryloyloxypropyloxy]phenyl)pyridin-5-ylcarbonyloxy]benzonitril;
3,4-bis[2-(4-[4-Acryloyloxybutyloxy]phenyl)pyridin-5-ylcarbonyloxy]benzonitril;
3,4-bis[2-(4-[5-Acryloyloxypentyloxy]phenyl)pyridin-5-ylcarbonyloxy]benzonitril;
3,4-bis[2-(4-[6-Acryloyloxyhexyloxy]phenyl)pyridin-5-ylcarbonyloxy]benzonitril;
3,4-bis[2-(4-[7-Acryloyloxyheptyloxy]phenyl)pyridin-5-ylcarbonyloxy]benzonitril;
3,4-bis[2-(4-[8-Acryloyloxyoctyloxy]phenyl)pyridin-5-ylcarbonyloxy]benzonitril;
3,4-bis[2-(4-[9-Acryloyloxynonyloxy]phenyl)pyridin-5-ylcarbonyloxy]benzonitril;
3,4-bis[2-(4-[10-Acryloyloxydecyloxy]phenyl)pyridin-5-ylcarbonyloxy]benzonitril;
3,4-bis[2-(4-[11-Acryloyloxyundecyloxy]phenyl)pyridin-5-ylcarbonyloxy]benzonitril;
3,4-bis[2-(4-[12-Acryloyloxydodecyloxy]phenyl)pyridin-5-ylcarbonyloxy]benzonitril;
Methyl 3,4-bis[2-(4-[3-acryloyloxypropyloxy]phenyl)pyridin-5-ylcarbonyloxy]benzoesäureester;
Ethyl 3,4-bis[2-(4-[3-acryloyloxypropyloxy]phenyl)pyridin-5-ylcarbonyloxy]benzoesäureester;
Propyl 3,4-bis[2-(4-[3-acryloyloxypropyloxy]phenyl)pyridin-5-ylcarbonyloxy]benzoesäureester;
3,4-bis[2-(4-[3-Acryloyloxypropyloxy]phenyl)pyridin-5-ylcarbonyloxy]toluol;
3,4-bis[2-(4-[3-Acryloyloxypropyloxy]phenyl)pyridin-5-ylcarbonyloxy]-1-ethylbenzol;
3,4-bis[2-(4-[3-Acryloyloxypropyloxy]phenyl)pyridin-5-ylcarbonyloxy]-1-propylbenzol.

### Beispiel 5

Eine Lösung aus 1,1 g 4-(5-[8-Acryloyloxyoctyloxy]pyrimidin-2-yl)benzylalkohol, 0,2 g 3, 4-Dihydroxybenzonitril, 0,5 g Azodicarbonsäurediethylester, 0,7 g Triphenylphosphin und 50 ml Tetrahydrofuran wird über Nacht gerührt und dann eingeengt. Der Rückstand wird mit 50 ml heissem Hexan aufgeschlämmt und filtriert. Das Filtrat wird eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,2 g 3,4-bis([4-(5-[8-Acryloyloxyoctyloxy]pyrimidin-2-yl)phenyl]methoxy)benzonitril.

Das als Ausgangsmaterial verwendete 4-(5-[8-Acryloyloxyoctyloxy]-pyrimidin-2-yl)benzylalkohol wird wie folgt hergestellt:
(a). Ein Gemisch von 0,3 g Natriumborhydrid und 30 ml Wasser wird tropfenweise bei 0^{o}C mit einer Lösung von 1,6 g 4-(5-[8-Acryloyloxyoctyloxy]pyrimidin-2-yl)benzaldehyd in 100 ml Dioxan versetzt. Das Reaktionsgemisch wird 60 Minuten bei 0^{o}C und dann 10 Minuten bei Raumtemperatur gerührt, auf 100 ml Dichlormethan gegossen und zweimal mit je 100 ml Wasser gewaschen. Die wässrigen Phasen werden vereinigt, zweimal mit je 50 ml Dichlormethan extrahiert. Die organischen Phasen werden vereinigt, zweimal mit je 100 ml Wasser gewaschen, über Magnesiumsulfat getrocknet, die Suspension filtriert und das Filtrat eingeengt. Dies ergibt 1,1 g 4-(5-[8-Acryloyloxyoctyloxy]pynmidin-2-yl)benzylalkohol.

In analoger Weise können folgende Verbindungen hergestellt werden:
3,4-bis([4-(5-[3-Acryloyloxypropyloxy]pyrimidin-2-yl)phenyl]methoxy)benzonitril;
3,4-bis([4-(5-[4-Acryloyloxybutoxy]pyrimidin-2-yl)phenyl]methoxy)benzonitril;
3,4-bis([4-(5-[5-Acryloyloxypentyloxy]pyrimidin-2-yl)phenyl]methoxy)benzonitril;
3,4-bis([4-(5-[6-Acryloyloxyhexyloxy]pyrimidin-2-yl)phenyl]methoxy)benzonitril;
3,4-bis([4-(5-[7-Acryloyloxyheptyloxy]pyrimidin-2-yl)phenyl]methoxy)benzonitril;
3,4-bis([4-(5-[8-Acryloyloxyoctyloxy]pyrimidin-2-yl)phenyl]methoxy)benzonitril;
3,4-bis([4-(5-[9-Acryloyloxynonyloxy]pyrimidin-2-yl)phenyl]methoxy)benzonitril;
3,4-bis([4-(5-[10-Acryloyloxydecyloxy]pyrimidin-2-yl)phenyl]methoxy)benzonitril;
3,4-bis([4-(5-[11-Acryloyloxyundecyloxy]pyrimidin-2-yl)phenyl]methoxy)benzonitril;
3,4-bis([4-(5-[12-Acryloyloxydodecyloxy]pyrimidin-2-yl)phenyl]methoxy)benzonitril;
Methyl 3,4-bis([4-(5-[12-acryloyloxydodecyloxy]pyrimidin-2-yl)phenyl]methoxy)benzoesäureester;
Ethyl 3,4-bis([4-(5-[12-acryloyloxydodecyloxy]pyrimidin-2-yl)phenyl]methoxy)benzoesäureester;
Propyl 3,4-bis([4-(5-[12-acryloyloxydodecyloxy]pyrimidin-2-yl)phenyl]methoxy)benzoesäureester;
3,4-bis([4-(5-[12-Acryloyloxydodecyloxy]pyrimidin-2-yl)phenyl]methoxy)toluol;
3,4-bis([4-(5-[12-Acryloyloxydodecyloxy]pyrimidin-2-yl)phenyl]methoxy)-1-ethylbenzol;
3,4-bis([4-(5-[12-Acryloyloxydodecyloxy]pyrimidin-2-yl)phenyl]methoxy)-1-propylbenzol;
3,4-bis([4-(5-[8-Acryloyloxyoctyloxy]pyridin-2-yl)phenyl]methoxy)benzonitril;
3,4-bis([2-(4-[3-Acryloyloxypropyloxy]phenyl)pyridin-5-yl]methoxy)benzonitril;
3,4-bis([2-(4-[4-Acryloyloxybutyloxy]phenyl)pyridin-5-yl]methoxy)benzonitril;
3,4-bis([2-(4-[5-Acryloyloxypentyloxy]phenyl)pyridin-5-yl]methoxy)benzonitril;
3,4-bis([2-(4-[6-Acryloyloxyhexyloxy]phenyl)pyridin-5-yl]methoxy)benzonitril;
3,4-bis([2-(4[7-Acryloyloxyheptyloxy]phenyl)pyridin-5-yl]methoxy)benzonitril;
3,4-bis([2-(4-[8-Acryloyloxyoctyloxy]phenyl)pyridin-5-yl]methoxy)benzonitril;
3,4-bis([2-(4-[9-Acryloyloxynonyloxy]phenyl)pyridin-5-yl]methoxy)benzonitril;
3,4-bis([2-(4-[10-Acryloyloxydecyloxy]phenyl)pyridin-5-yl]methoxy)benzonitril;
3,4-bis([2-(4-[11-Acryloyloxyundecyloxy]phenyl)pyridin-5-yl]methoxy)benzonitril;
3,4-bis([2-(4-[12-Acryloyloxydodecyloxy]phenyl)pyridin-5-yl]methoxy)benzonitril;
Methyl 3,4-bis([2-(4-[8-acryloyloxyoctyloxy]phenyl)pyridin-5-yl]methoxy)benzoesäureester;
Ethyl 3,4-bis([2-(4-[8-acryloyloxyoctyloxy]phenyl)pyridin-5-yl]methoxy)benzoesäureester;
Propyl 3,4-bis([2-(4-[8-acryloyloxyoctyloxy]phenyl)pyridin-5-yl]methoxy)benzoesäureester;
3,4-bis([2-(4-[8-Acryloyloxyoctyloxy]phenyl)pyridin-5-yl]methoxy)toluol;
3,4-bis([2-(4-[8-Acryloyloxyoctyloxy]phenyl)pyridin-5-yl]methoxy)-1-ethylbenzol;
3,4-bis([2-(4-[8-Acryloyloxyoctyloxy]phenyl)pyridin-5-yl]methoxy)-1-propylbenzol;
3,4-bis[4-(3-Acryloyloxypropyloxy)biphenyl-4'-methoxy]benzonitril;
3,4-bis[4-(4-Acryloyloxybutyloxy)biphenyl-4'-methoxy]benzonitril;
3,4-bis[4-(5-Acryloyloxypentyloxy)biphenyl-4'-methoxy]benzonitril;
3,4-bis[4-(6-Acryloyloxyhexyloxy)biphenyl-4'-methoxy]benzonitril;
3,4-bis[4-(7-Acryloyloxyheptyloxy)biphenyl-4'-methoxy]benzonitril;
3,4-bis[4-(8-Acryloyloxyoctyloxy)biphenyl-4'-methoxy]benzonitril;
3,4-bis[4-(9-Acryloyloxynonyloxy)biphenyl-4'-methoxy]benzonitril;
3,4-bis[4-(10-Acryloyloxydecyloxy)biphenyl-4'-methoxy]benzonitril;
3,4-bis[4-(11-Acryloyloxyundecyloxy)biphenyl-4'-methoxy]benzonitril;
3,4-bis[4-(12-Acryloyloxydodecyloxy)biphenyl-4'-methoxy]benzonitril;
Methyl 3,4-bis[4-(8-acryloyloxyoctyloxy)biphenyl-4'-methoxy]benzoesäureester;
Ethyl 3,4-bis[4-(8-acryloyloxyoctyloxy)biphenyl-4'-methoxy]benzoesäureester;
Propyl 3,4-bis[4-(8-acryloyloxyoctyloxy)biphenyl-4'-methoxy]benzoesäureester;
3,4-bis[4-(8-Acryloyloxyoctyloxy)biphenyl-4'-methoxy]toluol;
3,4-bis[4-(8-Acryloyloxyoctyloxy)biphenyl-4'-methoxy]-1-ethylbenzol;
3,4-bis[4-(8-Acryloyloxyoctyloxy)biphenyl-4'-methoxy]-1-propylbenzol;

### Beispiel 6

Zu einer Lösung von 2,0 g trans-4-(trans-4-[3-Acryloyloxypropyl]cyclohexyl)cyclohexancarbonsäure und 40 ml Tetrahydrofuran wird unter Rühren bei -25^{o}C 0,9 g Triethylamin und dann 0,5 g Methansulfochlorid zugetropft. Das Reaktionsgemisch wird 1 Stunde bei -25^{o}C gerührt, dann mit 0,28 g 3,4-Dihydroxybenzonitril und 0,05 g 4-(Dimethylamino)pyridin versetzt, über Nacht bei Raumtemperatur gerührt, auf 100 ml Wasser gegossen und dann dreimal mit je 50 ml Diethylether extrahiert. Die vereinigten organischen Phasen werden zweimal mit je 100 ml Wasser gewaschen, über Magnesiumsulfat getrocknet, filtriert und anschliessend eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,1 g 3,4-bis[trans-4-(trans-4-[3-Acryloyloxypropyl]cyclohexyl)cyclohexancarbonyloxy]benzonitril.

Die als Ausgangsmaterial verwendete trans-4-(trans-4-[3-Acryloyloxypropyl]cyclohexyl)cyclohexancarbonsäure wird wie folgt hergestellt:
(a). Eine Lösung von 0,3 ml 35-%iger Salzsäure und 10,0 g Methyl trans-4-(trans-4-[2-(1, 3-dioxan-2-yl)ethyl]cyclohexyl)cyclohexancarbonsäureester in 100 ml Toloul wird über Nacht bei Raumtemperatur gerührt, auf 100 ml Wasser gegossen. Die organischen Phase wird abgetrennt, zweimal mit je 100 ml Wasser gewaschen, über Magnesiumsulfat getrocknet, die Suspension filtriert und das Filtrat eingeengt. Dies ergibt 7,4 g trans-4-(trans-4-[2-Formylethyl]cyclohexyl)cyclohexancarbonsäuremethyl ester.
(b). Ein Gemisch von 1,4 g Natriumborhydrid und 140 ml Wasser wird tropfenweise bei 0^{o}C mit einer Lösung von 7,4 g trans-4-(trans-4-[2-Formylethyl]cyclohexyl)cyclohexancarbonsäuremethyl ester in 100 ml Dioxan versetzt. Das Reaktionsgemisch wird 60 Minuten bei 0^{o}C und dann 10 Minuten bei Raumtemperatur gerührt, auf 100 ml Dichlormethan gegossen und zweimal mit je 100 ml Wasser gewaschen. Die wässrigen Phasen werden vereinigt, zweimal mit je 50 ml Dichlormethan extrahiert. Die organischen Phasen werden vereinigt, zweimal mit je 100 ml Wasser gewaschen, über Magnesiumsulfat getrocknet, die Suspension filtriert und das Filtrat eingeengt. Dies ergibt 6,3 g trans-4-(trans-4-[3-Hydroxypropyl]cyclohexyl)cyclohexancarbonsäuremethylester.
(c). Eine Lösung von 2,0 g Kaliumhydroxid, 20 ml Wasser und 6,3 g trans-4-(trans-4-[3-Hydroxypropyl]cyclohexyl)cyclohexancarbonsäuremethylester in 100 ml Ethylalkohol wird 2 Stunden bei unter leichtem Reflux erwärmt, auf 100 ml Wasser gegossen und 25-%iger Salzsäure angesäuert. Der Niederschlag wird abfiltriert, portionenweise mit Wasser gewaschen und getrocknet. Dies ergibt 5,2 g trans-4-(trans-4-[3-Hydroxypropyl]cyclohexyl)cyclohexancarbonsäure.
(d). Zu einer Lösung von 5,2 g trans-4-(trans-4-[3-Hydroxpropyl]cyclohexyl)cyclohexancarbonsäure, 1,2 g Acrylsäure und 0,2 g 4-(Dimethylamino)pyridin in 25 ml Dichlormethan wird unter Rühren innert 5 Minuten 4,2 g N,N '-Dicyclohexylcarbodiimid gegeben. Das Reaktionsgemisch wird über Nacht weiter gerührt, filtriert und dann eingeengt. Chromatographie des Rückstandes an Kieselgel mit Hexan/Ethylacetat (Vol. 8:2) und Umkristallisation der gemäss Dünnschichtchromatographie reinen Fraktionen aus Ethylalkohol ergibt 4,2 g trans-4-(trans-4-[3-Acryloyloxypropyl]cyclohexyl)cyclohexancarbonsäure

In analoger Weise können folgende Verbindungen hergestellt werden:
3,4-bis[trans-4-(trans-4-[3-Acryloyloxypropyl]cyclohexyl)cyclohexancarbonyloxy]-2-fluorbenzonitril;
3,4-bis[trans-4-(trans-4-[3-Acryloyloxypropyl]cyclohexyl)cyclohexancarbonyloxy]-1-fluorbenzol;
3,4-bis[trans-4-(trans-4-[3-Acryloyloxypropyl]cyclohexyl)cyclohexancarbonyloxy]-1-chlorbenzol;
3,4-bis[trans-4-(trans-4-[3-Acryloyloxypropyl]cyclohexyl)cyclohexancarbonyloxy]-1-brombenzol;
3,4-bis[trans-4-(trans-4-[3-Acryloyloxypropyl]cyclohexyl)cyclohexancarbonyloxy]toluol;
3,4-bis[trans-4-(trans-4-[3-Acryloyloxypropyl]cyclohexyl)cyclohexancarbonyloxy]-1-ethylbenzol;
3,4-bis[trans-4-(trans-4-[3-Acryloyloxypropyl]cyclohexyl)cyclohexancarbonyloxy]-1-propylbenzol;
Methyl 3,4-bis[trans-4-(trans-4-[3-acryloyloxypropyl]cyclohexyl)cyclohexancarbonyloxy]benzoesäureester;
Ethyl 3,4-bis[trans-4-(trans-4-[3-acryloyloxypropyl]cyclohexyl)cyclohexancarbonyloxy]benzoesäureester;
Propyl 3,4-bis[trans-4-(trans-4-[3-acryloyloxypropyl]cyclohexyl)cyclohexancarbonyloxy]benzoesäureester;
3,4-bis[trans-4-(2-[trans-4-(3-Acryloyloxypropyl)cyclohexyl]ethyl)cyclohexancarbonyloxy]benzonitril;
Methyl 3,4-bis[trans-4(2-[trans-4-(3-acryloyloxypropyl)cyclohexyl]ethyl)cyclohexancarbonyloxy]benzoesäureester;
Ethyl 3,4-bis[trans-4-(2-[trans-4-(3-acryloyloxypropyl)cyclohexyl]ethyl)cyclohexancarbonyloxy]benzoesäureester;
3,4-bis[4-(trans-4-[3-acryloyloxypropyl]cyclohexyl)phenylcarbonyloxy]benzonitril;
3,4-bis[4-(trans-4-[3-Acryloyloxypropyl]cyclohexyl)phenylcarbonyloxy]-2-fluorbenzonitril;
3,4-bis[4-(trans-4-[3-Acryloyloxypropyl]cyclohexyl)phenylcarbonyloxy]-1-fluorbenzol;
3,4-bis[4-(trans-4-[3-Acryloyloxypropyl]cyclohexyl)phenylcarbonyloxy]-1-chlorbenzol;
3,4-bis[4-(trans-4-[3-Acryloyloxypropyl]cyclohexyl)phenylcarbonyloxy]-1-brombenzol;
3,4-bis[4-(trans-4-[3-Acryloyloxypropyl]cyclohexyl)phenylcarbonyloxy]toluol;
3,4-bis[4-(trans-4-[3-Acryloyloxypropyl]cyclohexyl)phenylcarbonyloxy]-1-ethylbenzol;
3,4-bis[4-(trans-4[3-Acryloyloxypropyl]cyclohexyl)phenylcarbonyloxy]-1-propylbenzol;
Methyl 3,4-bis[4-(trans-4-[3-acryloyloxypropyl]cyclohexyl)phenylcarbonyloxy]benzoesäureester;
Ethyl 3,4-bis[4-(trans-4-[3-acryloyloxypropyl]cyclohexyl)phenylcarbonyloxy]benzoesäureester;
Propyl 3,4-bis[4-(trans-4-[3-acryloyloxypropyl]cyclohexyl)phenylcarbonyloxy]benzoesäureester;
3,4-bis[4-(2-[trans-4-(3-Acryloyloxypropyl)cyclohexyl]ethyl)phenylcarbonyloxy]benzonitril;
Methyl 3,4-bis[4-(2-[trans-4-(3-acryloyloxypropyl)cyclohexyl]ethyl)phenylcarbonyloxy]benzoesäureester;
Ethyl 3,4-bis[4-(2-[trans-4-(3-acryloyloxypropyl)cyclohexyl]ethyl)phenylcarbonyloxy]benzoesäureester.

### Beispiel 7

Zu einer Lösung von 1.00 g Acrylsäure 6-[4'-[trans-4-(2-carboxy-ethyl)cyclohexyl]-biphenyl-4-yloxy]-hexylester, 0.127 g 3,4-Dihydroxybenzonitril und 0.255 g 4-Dimethylamino-pyridin (DMAP) in 30 ml Dichlormethan wird bei 0 °C langsam eine Lösung von 0.433 g N-Ethyl-N'-(3-dimethylaminopropy-carbodiimid-hydrochlorid (EDC) in 5 ml Dichlormethan zugetropft, über Nacht bei Raumtemperatur gerührt, auf 50 ml Wasser gegossen und dann dreimal mit je 25 ml Diethylether extrahiert. Die vereinigten organischen Phasen werden zweimal mit je 50 ml Waser gewaschen, über Magnesiumsulfat getrocknet, filtriert und anschliessend eingeengt. Chromatographische Reinigung des Rückstandes an Kieselgel mit Cyclohexan/Ethyl-acetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 0.48 g 3,4-Bis-[3-[trans-4-[4'-(6-acryloyloxy-hexyloxy)-biphenyl-4-yl]-cyclohexyl]-propionyloxy]-benzonitril.

Der als Ausgangsmaterial verwendete Acrylsäure 6-[4'-[trans-4-(2-carboxy-ethyl)-cyclohexyl]-biphenyl-4-yloxy]-hexylester wird wie folgt hergestellt:
(a). Eine Lösung von 5.238 g Dihydroxy-[4-(6-hydroxy-hexyloxy)-phenyl]-boran, 8.206 g Trifluormethansulfonsäure-4-[trans-4-(2-[1,3]dioxolan-2-yl-ethyl)-cyclohexyl]-phenylester, 6.368 g tri-Kaliumphosphat, 2.619 g Kaliumbromid und 0.578 g Tetrakis-triphenylphosphin)-palladium in 100 ml Dioxan wird über Nacht bei 85 °C unter Stickstoff gerührt. Nach dem Abkühlen verdünnt man die Lösung mit 100 ml Ether, wäscht dreimal mit je 50 ml Wasser, trocknet über Magnesiumsulfat, filtriert und engt ein. Der Rückstand wird säulenchromatographisch an Kieselgel mit Cyclohexam/Ethylacetat (Vol. 8:2) gereinigt. Dies ergibt 6.2 g 6-[4'-[trans-4-(2-[1,3]Dioxolan-2-yl-ethyl)cyclohexyl]-biphenyl-4-yloxy]-hexan-1-ol.
(b). Zu einer Losung von 6.2 g 6-[4'-[trans-4-(2-[1,3]Dioxolan-2-yl-ethyl)cyclohexyl]-biphenyl-4-yloxy]-hexan-1-ol, 1.48 g Acrylsäure und 1.67 g 4-Dimethylamino-pyridin in 50 ml Dichlormethan tropft man unter Rühren bei 0 - 5 °C innerhalb 5 Minuten 3.38 g Dicyclohexylcarbodiimid in 5 ml Dichlormethan zu. Das Reaktionsgemisch wird über Nacht weiter gerührt, filtriert und dann eingeengt. Chromatographie des Rückstandes an Kieselgel mit Cyclohexan/Ethylacetat (Vol. 8:2) ergibt 5.14 g Acrylsäure 6-[4'-[trans-4-(2-[1,3]dioxolan-2-yl-ethyl)-cyclohexyl]-biphenyl-4-yloxy]-hexylester.
(c). 5.14 g Acrylsäure 6-[4'-[trans-4-(2-[1,3]dioxolan-2-yl-ethyl)-cyclohexyl]-biphenyl-4-yloxy]-hexylester werden in 100 ml Toluol gelöst. Nach Zugabe von 50 ml Ameisensäure wird der Ansatz 4 Stunden bei Raumtemperatur gerührt. Dann trennt man die organische Phase ab und extrahiert die wässrige Phase zweimal mit 50 ml Toluol. Die vereinigten organischen Phasen werden zweimal mit 100 ml Wasser gewaschen, über Magnesiumsulfat getrocknet und eingeengt. Dies ergibt 4.55 g Acrylsäure-6-[4'-[trans-4-(3-oxo-propyl)-cyclohexyl]-biphenyl-4-yloxy]-hexylester.
(d). Eine Lösung von 4.55 g Acrylsäure- 6-[4'-[trans-4-(3-oxo-propyl)-cyclohexyl]-biphenyl-4-yloxy]-hexylester in 100 ml Aceton wird bei 5 °C tropfenweise mit 20 ml Jones' Reagenz versetzt. Das Gemisch wird über Nacht bei Raumtemperatur gerührt, auf 100 ml Wasser gegossen und dreimal mit 50 ml Ethylacetat extrahiert. Die organischen Phasen werden vereinigt, zweimal mit je 100 ml Wasser gewaschen, über Magnesiumsulfat getrocknet, filtriert und eigeengt. Dies ergibt 3.86 g Acrylsäure 6-[4'-[trans-4-(2-carboxy-ethyl)-cyclohexyl]-biphenyl-4-yloxy]-hexylester.

In analoger Weise können folgende Verbindungen hergestellt werden:
3,4-Bis-[3-[trans-4-[4'-(3-acryloyloxy-propyloxy)-biphenyl-4-yl]-cyclohexyl]-propionyloxy]-benzonitril
3,4-Bis-[3-[trans-4-[4'-(4-acryloyloxy-butyloxy)-biphenyl-4-yl]-cyclohexyl]-propionyloxy]-benzonitril
3,4-Bis-[3-[trans-4-[4'-(5-acryloyloxy-pentyloxy)-biphenyl-4-yl]-cyclohexyl]-propionyloxy]-benzonitril
3,4-Bis-[3-[trans-4-[4'-(6-acryloyloxy-hexyloxy)-biphenyl-4-yl]-cyclohexyl]-propionyloxy]-benzonitril
Methyl 3,4-Bis-[3-[trans-4-[4'-(6-acryloyloxy-hexyloxy)-biphenyl-4-yl]-cyclohexyl]-propionyloxy]-benzoesäureester
Ethyl 3,4-Bis-[3-[trans-4-[4'-(6-acryloyloxy-hexyloxy)-biphenyl-4-yl]-cyclohexyl]-propionyloxy]-benzoesäurester
Propyl 3,4-Bis-[3-[trans-4-[4'-(6-acryloyloxy-hexyloxy)-biphenyl-4-yl]-cyclohexyl]-propionyloxy]-benzoesäurester
Octyl 3,4-Bis-[3-[trans-4-[4'-(6-acryloyloxy-hexyloxy)-bipheny-4-yl]-cyclohexyl]-propionyloxy]-benzoesäureester
3,4-Bis-[3-[trans-4-[4'-(6-acryloyloxy-hexyloxy)-biphenyl-4-yl]-cyclohexyl]-propionyloxy]-benzol
3,4-Bis-[3-[trans-4-[4'-(6-acryloyloxy-hexyloxy)-biphenyl-4-yl]-cyclohexyl]-propionyloxy]-toluol
3,4-Bis-[3-[trans-4-[4'-(6-acryloyloxy-hexyloxy)-biphenyl-4yl]-cyclohexyl]-propionyloxy]-ethylbenzol
3,4-Bis-[3-[trans-4-[4'-(6-acryloyloxy-hexyloxy)-biphenyl-4-yl]-cyclohexyl]-propionyloxy]-propylbenzol

### Beispiel 8

Eine Lösung aus 1.50 g Acrylsäure 6-[4'-[trans-4-[3-(trifluormethansulfonyl)-propyl]-cyclohexyl]-biphenyl-4-yloxy]-hexyl ester und 0.155 g 3,4-Dihydroxy-benzonitril in 25 ml 1,2-Dimethoxyethan wird bei 0 °C portionweise mit 0.286 g Kalium-tertiär-butylat versetzt und anschliessend über Nacht bei 85 °C gerührt. Das abgekühlte Reaktionsgemisch wird auf 25 ml Wasser gegossen und dann dreimal mit 20 ml Ethylacetat extrahiert. Die vereinigten organischen Phasen werden zweimal mit 50 ml Wasser gewaschen, über Magnesiumsulfat getrocknet, filtriert und eingeengt. Chromatographische Reinigung des Rückstandes an Kieselgel mit Cyclohexan/Ethyl-acetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1.10 g 3,4-Bis-[3-[trans-4-[4'-(6-acryloyloxy-hexyloxy)-biphenyl-4-yl]-cyclohexyl]-propoxy]-benzonitril.

Der als Ausgangsmaterial verwendete Acrylsäure 6-[4'-[trans-4-[3-(trifluormethansulfonyl)-propyl]-cyclohexyl]-biphenyl-4-yloxy]-hexyl ester wird wie folgt hergestellt:
(a). Ein Gemisch von 0.123 g Natriumborhydrid und 10 ml Wasser wird bei 0 °C tropfenweise mit einer Lösung von 5.00 g Acrylsäure- 6-[4'-[trans-4-(3-oxo-propyl]-cyclohexyl]-biphenyl-4-yloxy]-hexylester in 50 ml Dioxan versetzt. Der Ansatz wird anschliessend 1 Stunde bei Raumtemperatur gerührt. Man giesst das Reaktionsgemisch auf 50 ml Wasser, trennt die Phasen und extrahiert die wässrige Phase zweimal mit je 25 ml Ethylacetat. Die vereinigten organischen Phasen werden zweimal mit je 50 ml Wasser gewaschen über Magnesiumsulfat getrocknet, filtriert und eingeengt. Chromatographische Reinigung des Rückstandes an Kieselgel mit Cyclohexan/Ethyl-acetat (Vol. 8:2) ergibt 4.77 g Acrylsäure- 6-[4'-[trans-4-(3-hydroxy-propyl)-cyclohexyl]-biphenyl-4-yloxy]-hexyl ester.
(b). Zu einer Lösung aus 4.77 g Acrylsäure 6-[4'-[trans-4-(3-hydroxy-propyl)-cyclohexyl]-biphenyl-4-yloxy]-hexylester und 1.32 g Lutidin in 100 ml Dichlormethan wird bei O °C 3.47 g Trifluormethansulfonsäureanhydrid gelöst in 10 ml Dichlormethan zugetropft. Das Reaktiongemisch wird 90 Minuten bei 0 °C gerührt und anschliessend auf 100 ml Wasser gegossen und zweimal mit 50 ml Dichlormethan extrahiert. Die vereinigten organischen Phasen werden zweimal mit je 50 ml Wasser gewaschen, über Magnesiumsulfat getrocknet und eingeengt. Chromatographische Reinigung des Rückstandes an Kieselgel mit Cyclohexan/Ethylacetat (Vol. 8:2) ergibt 5.09 g Acrylsäure 6-[4'-[trans-4-[3-(trifluormethansulfonyloxy)-propyl]-cyclohexyl]-biphenyl-4-yloxy]-hexylester.
In analoger Weise können folgende Verbindungen hergestellt werden:
3,4-Bis-[3-[trans-4-[4'-(3-acryloyloxy-propyloxy)-biphenyl-4-yl]-cyclohexyl]-propoxy]benzonitril
3,4-Bis-[3-[trans-4-[4'-(4-acryloyloxy-butyloxy)-biphenyl-4-yl]-cyclohexyl]-propoxy]benzonitril
3,4-Bis-[3-[trans-4-[4'-(5-acryloyloxy-pentyloxy)-biphenyl-4-yl]-cyclohexyl]-propoxy]benzonitril
3,4-Bis-[3-[trans-4-[4'-(6-acryloyloxy-hexyloxy)-biphenyl-4-yl]-cyclohexyl]-propoxy]benzonitril
Methyl 3,4-bis-[3-[trans-4-[4'-(6-acryloyloxy-hexyloxy)-biphenyl-4-yl]-cyclohexyl]-propoxy]benzoesäureester
Ethyl 3,4-bis-[3-trans-4-[4'-(6-acryloyloxy-hexyloxy)-biphenyl-4-yl]-cyclohexyl]-propoxy]benzoesäureester
Propyl 3,4-bis-[3-[trans-4-[4'-(6-acryloyloxy-hexyloxy)-biphenyl-4-yl]-cyclohexyl]-propoxy]benzoesäureester
Octyl 3,4-bis-[3-[trans-4-[4'-(6-acryloyloxy-hexyloxy)-biphenyl-4-yl]-cyclohexyl]-propoxy]benzoesäureester
3,4-bis-[3-[trans-4-[4'-(6-acryloyloxy-hexyloxy)-biphenyl-4-yl]-cyclohexy]-propoxy]benzol
3,4-bis-[3-[trans-4-[4'-(6-acryloyloxy-hexyloxy)-biphenyl-4-yl]-cyclohexyl]-propoxy]toluol
3,4-bis-[3-[trans-4-[4'-(6-acryloyloxy-hexyloxy)-biphenyl-4-yl]-cyclohexyl]-propoxy]ethylbenzol
3,4-bis-[3-[trans-4-[4'-(6-acryloyloxy-hexyloxy)-biphenyl-4-yl)-cyclohexyl-propoxy]propylbenzol.

### Beispiel 9

Zu einer Lösung von 1.00 g Acrylsäure-4-[2',3'-difluor-4''-(3-carboxy-propyl)-1,2':4',1''-terphenyl-4-yloxy]-butyl ester, 0.123 g 3,4-Dihydroxy-benzonitril und 0.247 g 4-Dimethylamino-pyridin (DMAP) in 30 ml Dichlormethan wird bei 0 °C langsam eine Lösung von 0.418 g N-Ethyl-N'-(3-dimethylaminopropyl)-carbodiimid-hydrochlorid (EDC) in 5 ml Dichlormethan zugetropft, über Nacht bei Raumtemperatur gerührt, auf 50 ml Wasser gegossen und dann dreimal mit je 25 ml Diethylether extrahiert. Die vereinigten organischen Phasen werden zweimal mit je 50 ml Waser gewaschen, über Magnesiumsulfat getrocknet, filtriert und anschliessend eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 0.39 g 3,4-Bis-[4-[4''-(4-acryloyloxy-butoxy)-2',3'-difluor-1,1':4',1''-terphenyl]-butyryloxy]-benzonitril.

Der als Ausgangsmaterial verwendete Acrylsäure 4-[2',3'-difluor-4''-(3-carboxy-propyl)-1,2':4',1''-terphenyl-4-yloxy]-butyl ester wird wie folgt hergestellt:
(a). Zu einer Lösung von 5.80 g 2-[3-(2',3'-Difluor-biphenyl-4-yl)-propyl]-[1,3]dioxolan in 80 ml trockenem Tetrahydrofuran unter Stickstoff tropft man bei -78 °C langsam 13.0 ml 1.6 molare Lösung von Butyl-Lithium in Hexan zu und lässt 2.5 Stunden bei dieser Temperatur rühren. Dann tropft man eine Lösung von 4.32 g Trimethylborat in 10 ml Tetrahydrofuran zu. Man lässt die Lösung langsam erwärmen und rührt über Nacht bei Raumtemperatur. Dann setzt man 50 ml 10%ige Salzsäure zu, rührt eine Stunde, trennt die Phasen und extrahiert zweimal mit 25 ml Ether. Die vereinigten organischern Phasen werden zweimal mit 50 ml Wasser gewaschen, über Magnesiumsulfat getrocknet, filtriert und eingeengt. Dies ergibt 5.3 g [2,3-Difluor-4'-(4-oxo-butyl)-bipheny-4-yl]-dihydroxy-boran.
(b). Eine Lösung von 5.30 g [2,3-Difluor-4'-(4-oxo-butyl)-biphenyl-4-yl]-dihydroxy-boran, 6.18 g Acrylsäure 3-(bromo-phenoxy)-propyl ester, 0.50 g Tetrakis-(triphenylphosphin)-palladium und 17.5 ml 2M Natriumcarbonat-Lösung in 100 ml 1,2-Dimethoxyethan wird über Nacht bei 85 °C unter Stickstoff gerührt. Nach dem Abkühlen verdünnt man die Lösung mit 100 ml Ether, wäscht dreimal mit je 50 ml Wasser, trocknet über Magnesiumsulfat, filtriert und engt ein. Der Rüchstand wird säulenchromatographisch an Kieselgel mit Cyclo-hexan/Ethylacetat (Vol. 8:2) gereinigt. Dies ergibt 5.55 g Acrylsäure 4-[2',3'-difluor-4''-(4-oxo-butyl)-1,2':4',1''-terphenyl-4-yloxy]- butylester.
(c). Eine Lösung von 5.55 g Acrylsäure 4-[2',3'-difluor-4''-(4-oxo-butyl)-1,2':4',1''-terphenyl-4-yloxy]- butyl ester in 100 ml Aceton wird bei 5 °C tropfenweise mit 20 ml Jones' Reagenz versetzt. Das Gemisch wird über Nacht bei Raumtemperatur gerührt, auf 100 ml Wasser gegossen, dreimal mit 50 ml Ethylacetat extrahiert. Die organischen Phasen werden vereinigt, zweimal mit je 100 ml Wasser gewaschen, über Magnesiumsulfat getrocknet, filtriert und eingeengt. Dies ergab 5.29 g Acrylsäure 4-[2',3'-difluor-4''-(3-carboxy-propyl)-1,2':4',1''-terphenyl-4-yloxy]-butyl ester.

In analoger Weise können folgende Verbindungen hergestellt werden:
3,4-Bis-[4-[4''-(3-acryloyloxy-propoxy)-2',3'-difluor-1,1':4',1''-teryhenyl]-butyryloxy]-benzonitril
3,4-Bis-[4-[4''-(5-acryloyloxy-pentoxy)-2'3'-difluor-1,1':4',1''-terphenyl]-butyryloxy]benzonitril
3,4-Bis-[4-[4''-(6-acryloyloxy-hexoxy)-2',3'-difluor-1,1':4',1''-terphenyl]-butyryloxy]benzonitril
Methyl 3,4-bis-[4-[4''-(4-acryloyloxy-butoxy)-2'3'-difluor-1,1':4'1''-terphenyl]-butyryloxy]-benzoesäureester
Ethyl 3,4-bis-[4-[4''-(4-acryloyloxy-butoxy)-2',3'-difluor-1,1':4',1''-terphenyl]-butyryloxy]-benzoesäureester
Propyl 3,4-bis-[4-[4''-(4-acryloyloxy-butoxy)-2',3'-difluor-1,1':4',1''-terphenyl]-butyryloxy]-benzoesäureester
Octyl 3,4-bis-[4-[4''-(4-acryloyloxy-butoxy)-2',3'-difluor-1,1':4'1''-terphenyl]-butyryloxy]-benzoesäureester
3,4-bis-[4-[4''-(4-acryloyloxy-butoxy)-2'3'-difluor-1,1':4'1''-terphenyl]-butyryloxy]-benzol
3,4-bis-[4-[4''-(4-acryloyloxy-butoxy)-2',3'-difluor-1,1':4',1''-terphenyl]-butyryloxy]-toluol
33,4-bis-[4-[4''-(4-acryloyloxy-butoxy)-2'3'-difluor-1,1':4'1''-terphenyl]-butyryloxy]-ethylbenzol
3,4-bis-[4-[4''-(4-acryloyloxy-butoxy)-2',3'-difluor-1,1':4',1''-terphenyl]-butyryloxy]-propylbenzol

### Beispiel 10

Eine Lösung aus 1.50 g Acrylsäure 4-[2',3'-difluor-4''-[4-(trifluormethansulfonyloxy)-butyl]-1,2':4',1''-terphenyl-4-yloxy]-butyl ester und 0.156 g 3,4-Dihydroxybenzonitril in 25 ml 1,2-Dimethoxyethan wird bei 0 °C portionweise mit 0.292 g Kalium-tertiär-butylat versetzt und anschliessend über Nacht bei 85 °C gerührt. Das abgekühlte Reaktionsgemisch wird auf 25 ml Wasser gegossen und dann dreimal mit 20 ml Ethylacetat extrahiert. Die vereinigten organischen Phasen werden zweimal mit 50 ml Wasser gewaschen, über Magnesiumsulfat getrocknet, filtriert und eingeengt. Chromatographische Reinigung des Rückstandes an Kieselgel mit Cyclohexan/Ethyl-acetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 0.65 g 3,4-Bis-[4-[4-(4-acryloyloxy-butoxy)-2',3'-difluor-1,1':4',1''-terphenyl]-butoxy]benzonitril.

Der als Ausgangsmaterial verwendete Acrylsäure 4-[2',3'-difluor-4''-[4-(trifluormethansulfonyloxy)-butyl]-1,2':4',1''-terphenyl-4-yloxy]-butyl ester wird wie folgt hergestellt:
(a). Ein Gemisch von 0.119 g Natriumborhydrid und 10 ml Wasser wird bei 0 °C tropfenweise mit einer Lösung von 5.00 g Acrylsäure 4-[2',3'-difluor-4''-(4-oxo-butyl)-1,2':4',1''-terphenyl-4-yloxy]-butyl ester in 50 ml Dioxan versetzt. Der Ansatz wird anschliessend 1 Stunde bei Raumtemperatur gerührt. Man giesst das Reaktionsgemisch auf 50 ml Wasser, trennt die Phasen und extrahiert die wässrige Phase zweimal mit je 25 ml Ethylacetat. Die vereinigten organischen Phasen werden zweimal mit je 50 ml Wasser gewaschen über Magnesiumsulfat getrocknet, filtriert und eingeengt. Chromatographische Reinigung des Rückstandes an Kieselgel mit Cyclohexan/Ethyl-acetat (Vol. 8:2) ergibt 4.49 g Acrylsäure 4-[2',3'-difluor-4''-(4-hydroxy-butyl)-1,2':4',1''-terphenyl-4-yloxy]-butyl ester.
(b). Zu einer Lösung aus 4.40 g Acrylsäure 4-[2',3'-difluor-4''-(4-hydroxy-butyl)-1,2':4',1''-terphenyl-4-yloxy]-butyl ester und 1.18 g Lutidin in 100 ml Dichlormethan wird bei O °C 3.10 g Trifluormethansulfonsäureanhydrid gelöst in 10 ml Dichlormethan zugetropft. Das Reaktiongemisch wird 90 Minuten bei 0 °C gerührt und anschliessend auf 100 ml Wasser gegossen und zweimal mit 50 ml Dichlormethan extrahiert. Die vereinigten organischen Phasen werden zweimal mit je 50 ml Wasser gewaschen, über Magnesiumsulfat getrocknet und eingeengt. Chromatographische Reinigung des Rückstandes an Kieselgel mit Cyclohexan/Ethylacetat (Vol. 8:2) ergibt 4.20 g Acrylsäure- 4-[2',3'-difluor-4''-[4-(trifluormethansulfonyloxy)-butyl]-1,2':4',1''-terphenyl-4-yloxy]-butyl ester.

In analoger Weise können folgende Verbindungen hergestellt werden:
3,4-Bis-[4-[4-(3-acryloyloxy-propoxy)-2',3'-difluor-1,1':4',1''-terphenyl]-butoxy]benzonitril
3,4-Bis-[4-[4-(5-acryloyloxy-pentoxy)-2',3'-difluor-1,1':4',1''-terphenyl]-butoxy]benzonitril
3,4-Bis-[4-[4-(6-acryloyloxy-hexoxy)-2',3'-difluor-1,1':4',1''-terphenyl]-butoxy]benzonitril
Methyl 3,4-bis-[4-[4-(4-acryloyloxy-butoxy)-2',3'-difluor-1,1':4',1''-terphenyl]-butoxy]benzoesäureester
Ethyl 3,4-bis-[4-[4-(4-acryloyloxy-butoxy)-2',3'-difluor-1,1':4',1''-terphenyl]-butoxy]benzoesäureester
Propyl 3,4-bis-[4-[4-(4-acryloyloxy-butoxy)-2',3'-difluor-1,1':4',1''-terphenyl]-butoxy]benzoesäureester
Octyl 3,4-bis-[4-[4-(4-acryloyloxy-butoxy)-2',3'-difluor-1,1':4',1''-terphenyl]-butoxy]benzoesäureester
3,4-bis-[4-[4-(4-acryloyloxy-butoxy)-2',3'-difluor-1,1':4',1''-terphenyl]-butoxy]benzol
3,4-bis-[4-[4-(4-acryloyloxy-butoxy)-2',3'-difluor-1,1':4',1''-terphenyl]-butoxy]ethylbenzol
3,4-bis-[4-[4-(4-acryloyloxy-butoxy)-2',3'-difluor-1,1':4',1''-terphenyl]-butoxy]propylbenzol

## Patentansprüche

1. Verbindungen der allgemeinen Formel I 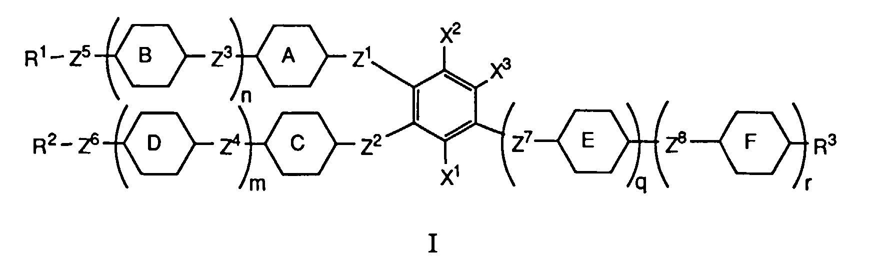 worin
Ringe A - F je Pyridin-2,5-diyl, Pyrimidin-2,5-diyl, trans-1,4-Cyclohexylen, trans-1,3-Dioxan-2,5-diyl oder unabhängig voneinander gegebenenfalls mit Halogen, Methyl und/oder Cyano substituiertes 1,4-Phenylen;
Z¹, Z² und Z⁷ unabhängig voneinander ⁻CH₂-(CH₂)ₛ-, -(CH₂)ₛO-, -O(CH₂)ₛ-, -COO-, -OOC-, -(CH₂)ₛCOO- ⁻ oder -(CH₂)ₛOOC-;
Z³, Z⁴ und Z⁸ unabhängig voneinander eine Einfachbindung, -CH₂CH₂-, -CH₂O-, -OCH₂-, -COO-, -OOC-, -(CH₂)₄-, -O(CH₂)₃- oder -(CH₂)₃O-;
Z⁵ und Z⁶ -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, oder -NHCH₂(Si[(CH₃)₂]O)ₛSi[(CH₃)₂]CH₂NH-;
Y Wasserstoff oder Fluor;
m, n, q und r unabhängig voneinander 0, 1 oder 2;
s eine ganze Zahl von 1 bis 16;
R¹ und R² vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 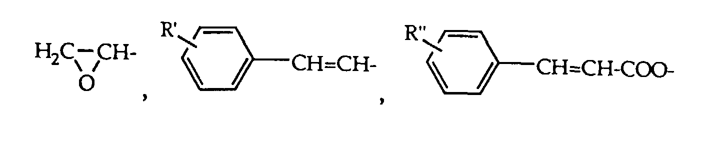
Ph Phenyl;
R' niederes Alkyl;
R'' Methyl, Methoxy, Cyano oder Halogen bedeuten, mit der Massgabe, dass R¹-Z⁵ und R²-Z⁶ keine -O-O- oder -N-O- Gruppen enthalten;
R³ Wasserstoff, Halogen, Cyano, oder eine gegebenenfalls mit Methoxy, Cyano und/oder Halogen substituierte Alkyl-, Alkoxy-, Alkoxycarbonyl- oder Alkanoyloxy-Gruppe; und
X¹, X² und X³ unabhängig Wasserstoff, Halogen, Cyano oder Niederalkyl bedeuten,
wobei für m=2 und/oder n=2 die beiden Ringe B und D gleich oder verschieden sein können.

2. Verbindungen gemäss Anspruch 1 der Formel I 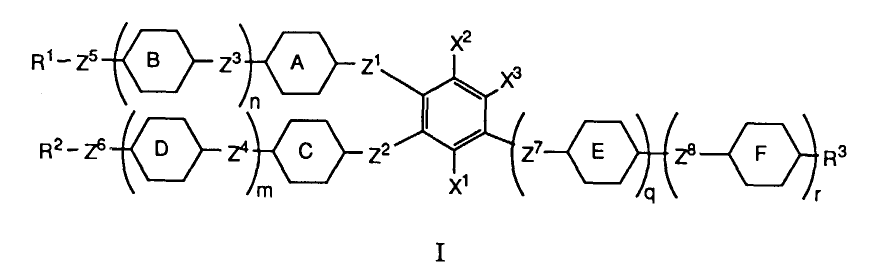 worin
Ringe A - F je Pyridin-2,5-diyl, Pyrimidin-2,5-diyl, trans-1,4-Cyclohexylen, trans- 1,3-Dioxan-2,5-diyl oder unabhängig voneinander gegebenenfalls mit Halogen, Methyl und/oder Cyano substituiertes 1,4-Phenylen;
Z¹, Z² und Z⁷ unabhängig voneinander ⁻CH₂-CH₂-, -CH₂-O-, -COO-, -OOC-, -(CH₂)₄- oder -(CH₂)₃O-;
Z³, Z⁴ und Z⁸ unabhängig voneinander eine Einfachbindung, -CH₂CH₂-, -CH₂O-, -OCH₂-, -COO-, -OOC-, -(CH₂)₄-, -O(CH₂)₃- oder -(CH₂)₃O-;
Z⁵ und Z⁶ -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, oder -NHCH₂(Si[(CH₃)₂]O)ₛSi[(CH₃)₂]CH₂NH-;
Y Wasserstoff oder Fluor;
m, n, q und r unabhängig voneinander 0 oder 1;
s eine ganze Zahl von 1 bis 16;
R¹ und R² vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 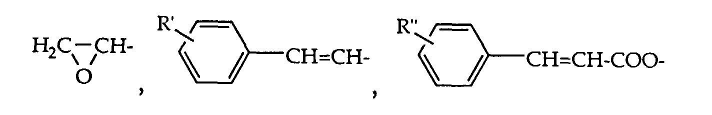
Ph Phenyl;
R' niederes Alkyl;
R'' Methyl, Methoxy, Cyano oder Halogen bedeuten, mit der Massgabe, dass R¹-Z⁵ und R²-Z⁶ keine -O-O- oder -N-O- Gruppen enthalten;
R³ Wasserstoff, Halogen, Cyano, oder eine gegebenenfalls mit Methoxy, Cyano und/oder Halogen substituierte Alkyl-, Alkoxy- oder Alkanoyloxy-Gruppe; und
X¹, X² und X³ unabhängig Wasserstoff, Halogen, Cyano oder Niederalkyl bedeuten.

3. Verbindungen nach Anspruch 1 oder 2, worin die vernetzbaren Reste R¹ und R² unabhangig voneinander CH₂=CH-COO-, CH₂=C(CH₃)-COO-, CH₂=CH-O-, 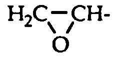 bedeuten.

4. Verbindungen nach einem der Ansprüche 1 bis 3, worin X¹, X² und X³ Wasserstoff, Fluor, Chlor, Cyano und Methyl bedeuten.

5. Verbindungen nach einem der Ansprüche 1 bis 4 der allgemeinen Formel IA 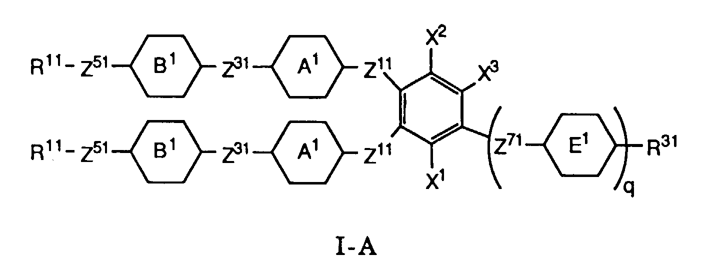 worin
A¹, B¹ und E¹ unabhangig voneinander gegebenenfalls mit Fluor substituiertes 1,4-Phenylen, Pyridin-2,5-diyl, Pyrimidin-2,5-diyl oder trans-1,4-Cyclohexylen;
Z¹¹ -CH₂CH₂-, -CH₂O-, -COO-, -OOC-, -(CH₂)₄- oder -(CH₂)₃O-;
Z³¹ eine Einfachbindung, -CH₂O-, -COO- oder -OOC-;
Z⁵¹ -(CH₂)_{s'}-, -(CH₂)_{s'}O-, -(CH₂)_{s'}COO- oder -(CH₂)_{s'}OOC-;
Z⁷¹ -CH₂O- oder -COO-;
s' eine ganze Zahl von 3 bis 12;
R¹¹ CH₂=CH-COO-, CH₂=C(CH₃)-COO-, CH₂=CH-O-, 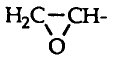
R³¹ Halogen, Cyano, niederes Alkyl oder Alkoxycarbonyl
q 0 oder 1 bedeuten und
X¹, X² und X³ die in Anspruch 1 angegebene Bedeutung haben.

6. Verbindungen nach Anspruch 5 der Formel Ia-c 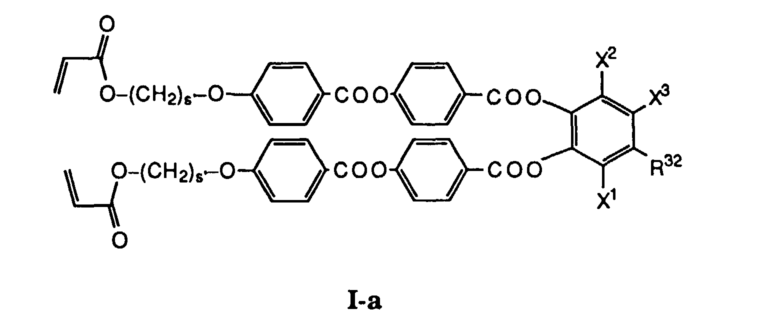 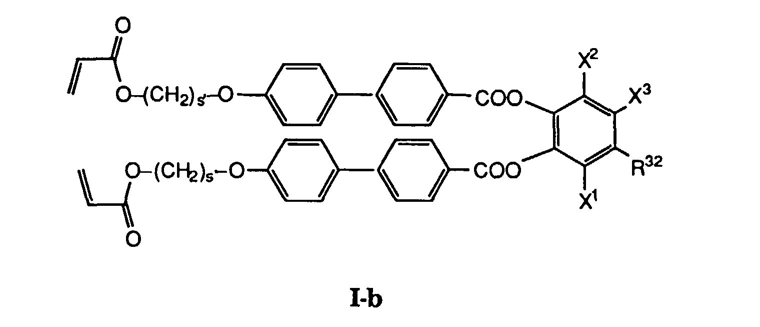 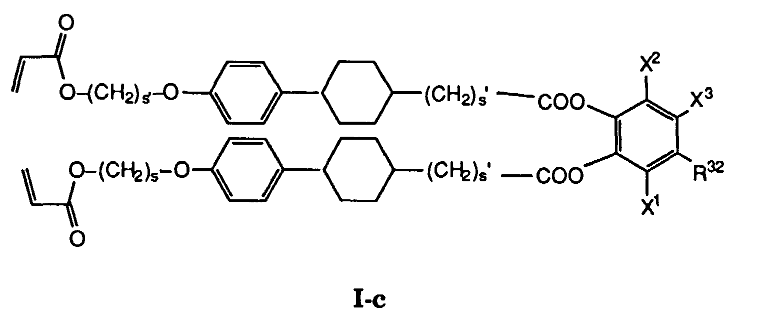 worin R³² Cyano oder Alkoxycarbonyl; X¹, X² und X³ Wasserstoff bedeutet; und s' die in Anspruch 5 angegebene Bedeutung hat.

7. Verbindungen nach einem der Ansprüche 1 bis 4 der allgemeinen Formel I-B 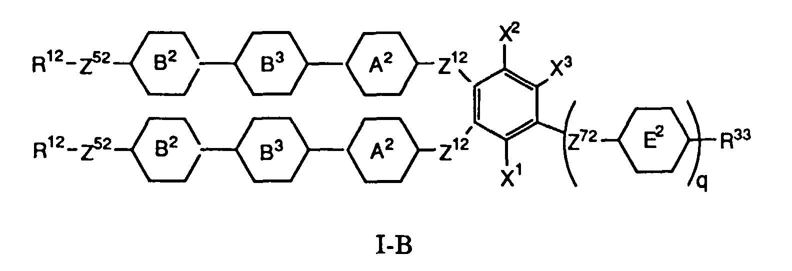 worin
A², B², B³und E² unabhängig voneinander gegebenenfalls mit Fluor substituiertes 1,4-Phenylen oder trans-1,4-Cyclohexylen;
Z¹² (CH₂)₂COO-, (CH₂)_{s'}COO- oder -(CH₂)_{s'}O-;
Z⁵² -(CH₂)_{s'}-, -(CH₂)_{s'}O-, -(CH₂)_{s'}COO- oder -(CH₂)_{s'}OOC-;
Z⁷² -CH₂O- oder -COO-;
s' eine ganze Zahl von 3 bis 12;
R¹² CH₂=CH-COO-, CH₂=C(CH₃)-COO-, CH₂=CH-O-, 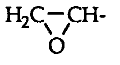
R³³ Halogen, Cyano oder Alkoxycarbonyl bedeuten.
q 0 oder 1 bedeuten und
X¹, X² und X³ die unter Anspruch 1 angegebene Bedeutung haben.

8. Verbindungen nach Anspruch 7 der Formel Id-g 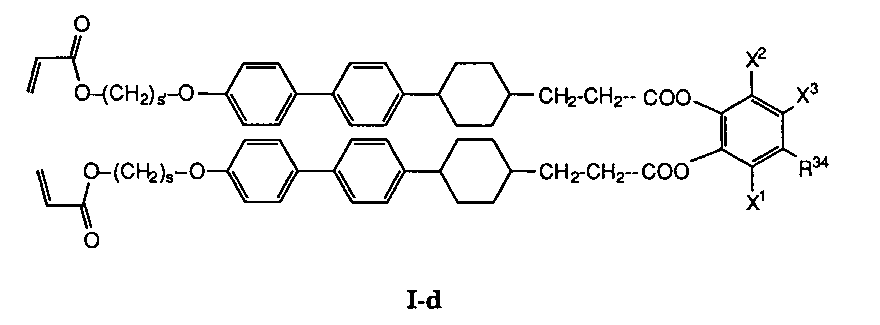 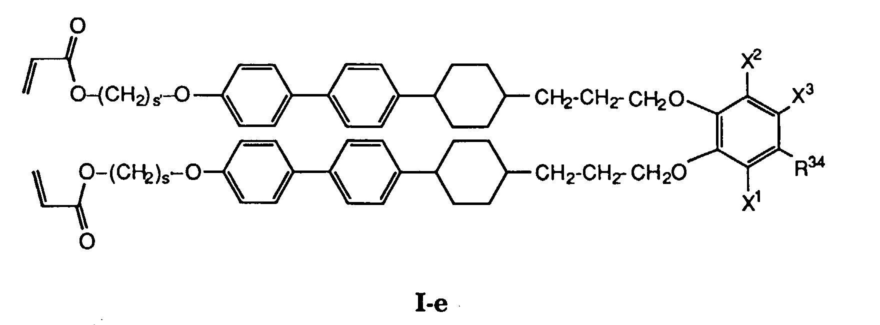 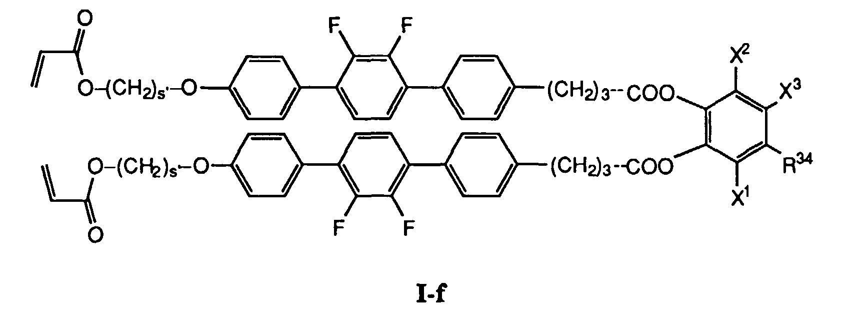 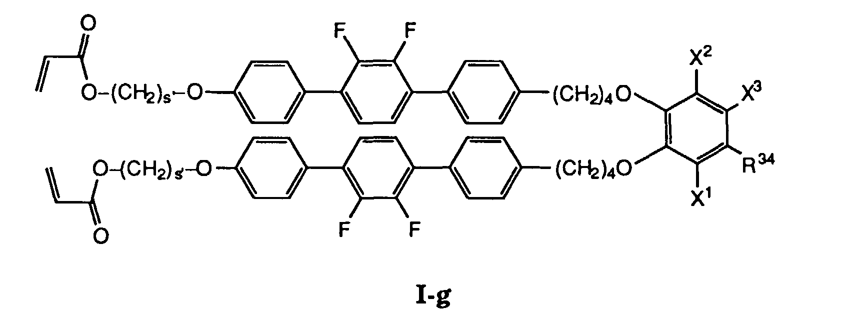 worin R³⁴ Cyano oder Alkoxycarbonyl; X¹, X² und X³ Wasserstoff bedeutet; und s' die in Anspruch 7 angegebene Bedeutung hat.

9. Vernetzbare, flüssigkristalline Gemische bestehend aus mindestens 2 Komponenten, wovon mindestens eine Komponente eine Verbindung der in Anspruch 1 definierten Formel I ist.

10. Vernetzbare, flüssigkristalline Gemische gemäss Anspruch 9, dadurch gekennzeichnet, dass sie neben einer oder mehreren Verbindungen der Formel I, eine oder mehrere Verbindungen aus der Gruppe der Formeln 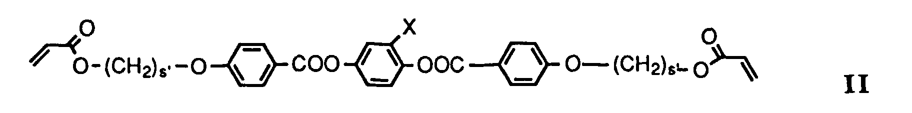 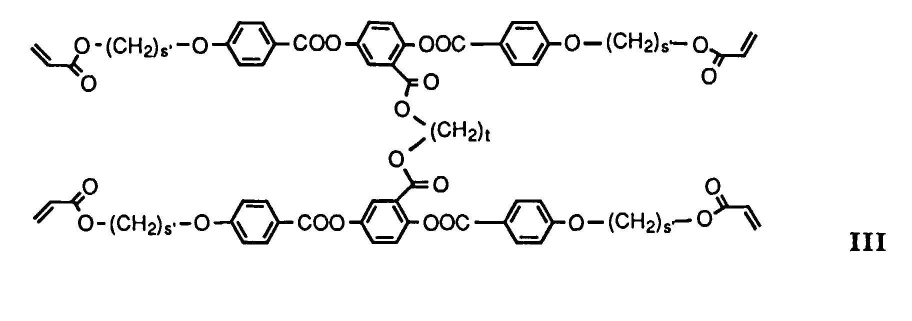 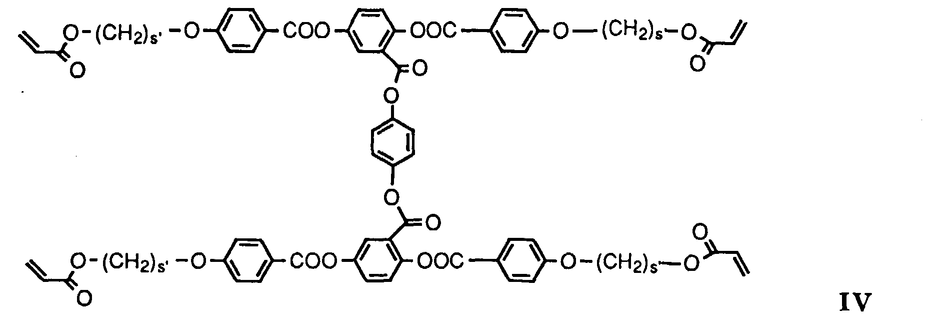 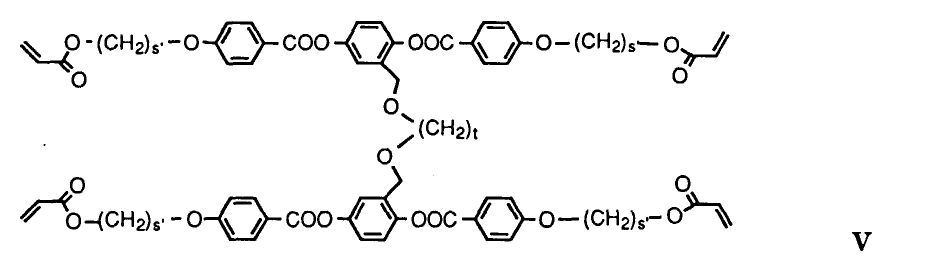 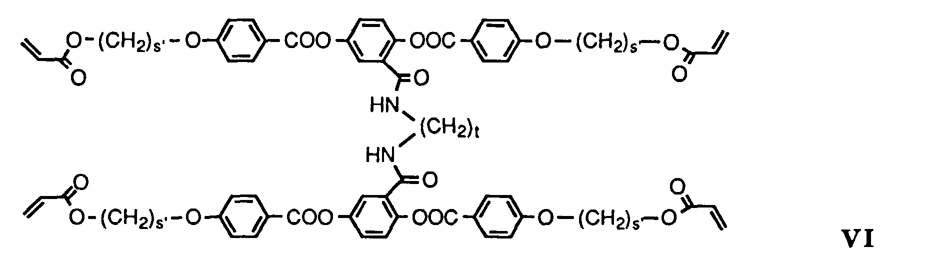 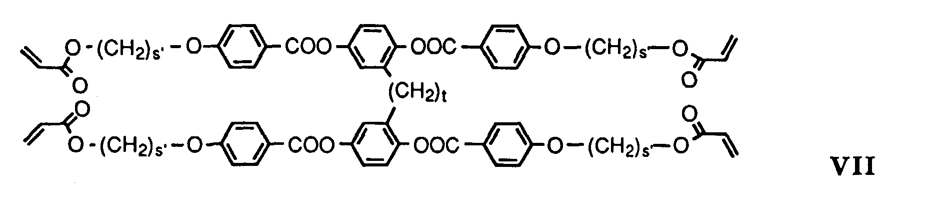 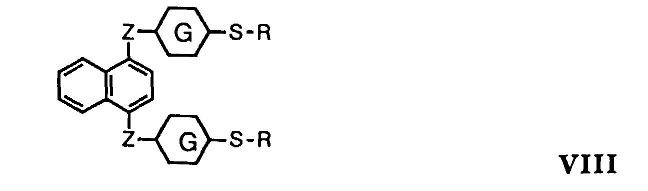 und 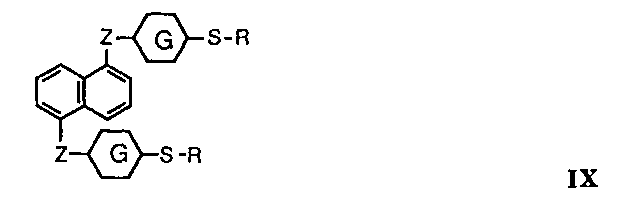 worin
X Wasserstoff, Fluor, Chlor, Brom oder Methyl;
s' eine ganze Zahl von 3 bis 12;
t eine ganze Zahl von 2 bis 12;
Z -OCH₂- oder -OOC-;
G 1,4-Phenylen oder 2- bzw. 3-Fluor-1,4-phenylen;
S -(CH₂)_{s'} - oder -(CH₂)_{s'}O-; und
R CH₂=CH-COO-, CH₂=C(CH₃)-COO-, CH₂=CH-O- oder 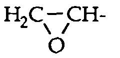 bedeuten.

11. Verwendung von Verbindungen gemäss einem der Ansprüche 1 bis 8 in ihrem vernetzten Zustand für optische Bauelemente.

12. Verwendung von vernetzbaren flüssigkristallinen Gemischen gemäss einem der Ansprüche 9 oder 10 in ihrem vernetzten Zustand für optische Bauelemente.

## Claims

1. Compounds of the general formula I 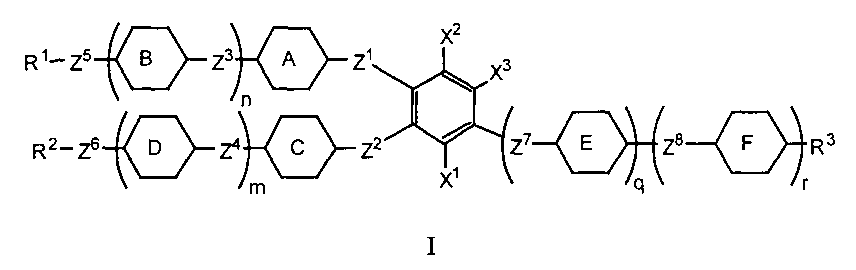 wherein
rings A - F each signify pyridine-2,5-diyl, pyrimidine-2,5-diyl, trans-1,4-cyclohexylene, trans-1,3-dioxane-2,5-diyl or independently of one another 1,4-phenylene optionally substituted with halogen, methyl and/or cyano;
Z¹, Z² and Z⁷ independently of one another signify -CH₂-(CH₂)ₛ-, -(CH₂)ₛO-, -O(CH₂)ₛ-, -COO-, -OOC-, -(CH₂)ₛCOO- ⁻ or -(CH₂)ₛOOC-;
Z³, Z⁴ and Z⁸ independently of one another signify a single bond, -CH₂CH₂-, -CH₂O-, -OCH₂-, -COO-, -OOC-, -(CH₂)₄-, -O(CH₂)₃- or -(CH₂)₃O-;
Z⁵ and Z⁶ signify -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, or -NHCH₂(Si[(CH₃)₂]O)ₛSi[(CH₃)₂]CH₂NH-;
Y signifies hydrogen or fluorine;
m, n, q and r independently of one another signify 0, 1 or 2;
s signifies a whole number of 1 to 16;
R¹ and R² signify cross-linkable groups of the structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 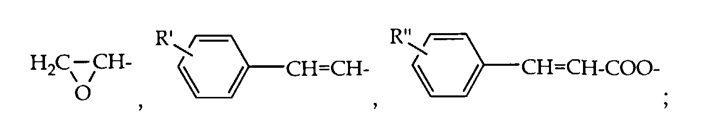
Ph signifies phenyl;
R' signifies lower alkyl;
R" signifies methyl, methoxy, cyano or halogen, with the proviso that R¹-Z⁵ and R²-Z⁶ contain no -O-O- or -N-O-groups;
R³ signifies hydrogen, halogen, cyano or an alkyl, alkoxy, alkoxycarbonyl or alkanoyloxy group optionally substituted with methoxy, cyano and/or halogen; and
X¹, X² and X³ independently signify hydrogen, halogen, cyano or lower alkyl,
wherein the two rings B and D can be the same or different when m is 2 and/or n is 2.

2. Compounds according to claim 1 of formula I 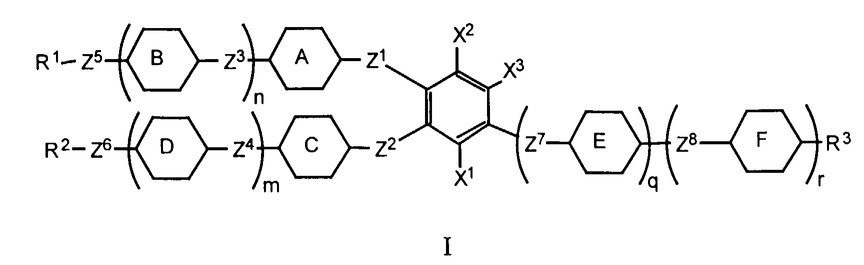 wherein
rings A - F each signify pyridine-2,5-diyl, pyrimidine-2,5-diyl, trans-1,4-cyclohexylene, trans-1,3-dioxane-2,5-diyl or independently of one another 1,4-phenylene optionally substituted with halogen, methyl and/or cyano;
Z¹, Z² and Z⁷ independently of one another signify -CH₂-(CH₂)-, -(CH₂)-O-, -COO-, -OOC-, -(CH₂)₄- or -(CH₂)₃O-;
Z³, Z⁴ and Z⁸ independently of one another signify a single bond, -CH₂CH₂-, -CH₂O-, -OCH₂-, -COO-, -OOC-, -(CH₂)₄-, -O(CH₂)₃- or -(CH₂)₃O-;
Z⁵ and Z⁶ signify -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, or -NHCH₂(Si[(CH₃)₂O)ₛSi[(CH₃)₂]CH₂NH-;
Y signifies hydrogen or fluorine;
m, n, q and r independently of one another signify 0 or 1;
s signifies a whole number of 1 to 16;
R¹ and R² signify cross-linkable groups of the structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 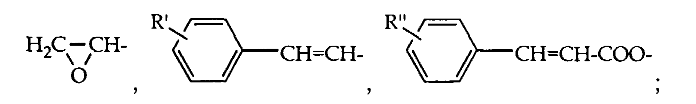
Ph signifies phenyl;
R' signifies lower alkyl;
R" signifies methyl, methoxy, cyano or halogen, with the proviso that R¹-Z⁵ and R²-Z⁶ contain no -O-O- or -N-O-groups;
R³ signifies hydrogen, halogen, cyano or an alkyl, alkoxy or alkanoyloxy group optionally substituted with methoxy, cyano and/or halogen; and
X¹, X² and X³ independently signify hydrogen, halogen, cyano or lower alkyl.

3. Compounds according to claim 1 or 2, wherein the cross-linkable residues R¹ and R² independently of one another signify CH₂=CH-COO-, CH₂=C(CH₃)-COO-, CH₂=CH-O- or

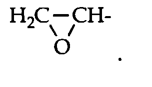

4. Compounds according to any one of claims 1 to 3, wherein X¹, X² and X³ signify hydrogen, fluorine, chlorine, cyano and methyl.

5. Compounds according to any one of claims 1 to 4 of general formula IA 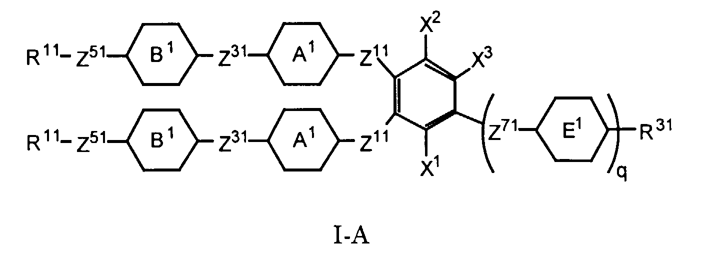 wherein
A¹, B¹ and E¹ independently of one another signify 1,4-phenylene optionally substituted with fluorine, pyridine-2,5-diyl, pyrimidine-2,5-diyl or trans-1,4-cyclohexylene;
Z¹¹ signifies -CH₂CH₂-, -CH₂O-, -COO-, -OOC-, -(CH₂)₄- or -(CH₂)₃O-;
Z³¹ signifies a single bond, -CH₂O-, -COO- or -OOC-;
Z⁵¹ signifies -(CH₂)_{s'}-, -(CH₂)_{s'}O-, -(CH₂)_{s'}COO- or -(CH₂)_{s'}OOC-;
Z⁷¹ signifies -CH₂O- or -COO-;
s' signifies a whole number of 3 to 12;
R¹¹ signifies CH₂=CH-COO-, CH₂=C(CH₃)-COO-, CH₂=CH-O- or 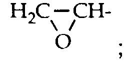
R³¹ signifies halogen, cyano, lower alkyl or alkoxycarbonyl;
q signifies 0 or 1 and
X¹, X² and X³ have the significance given in claim 1.

6. Compounds according to claim 5 of formulae Ia-c 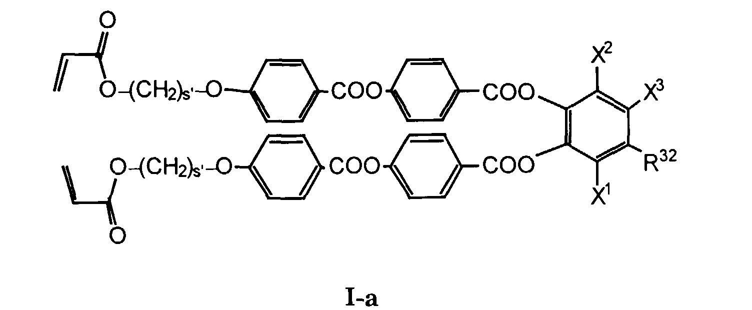 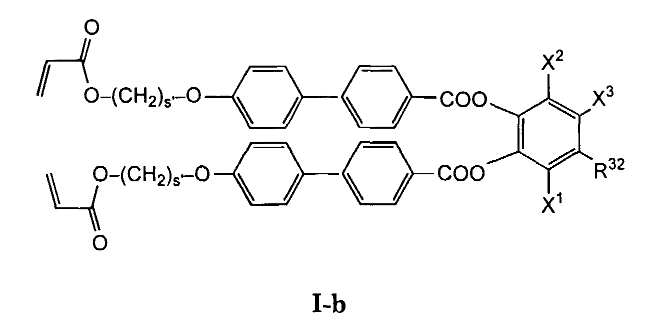 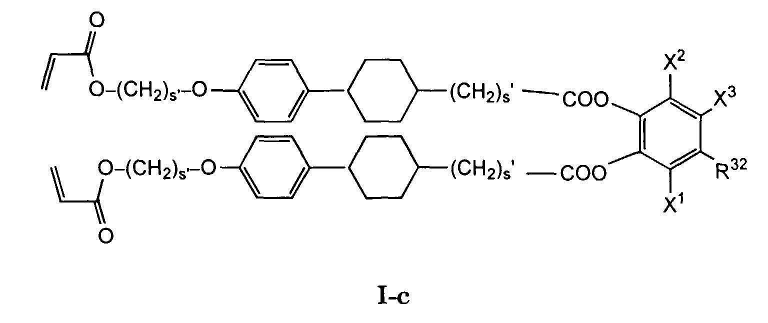 wherein R³² signifies cyano or alkoxycarbonyl; X¹, X² and X³ signify hydrogen; and s' has the significance given in claim 5.

7. Compounds according to any one of claims 1 to 4 of general formula I-B 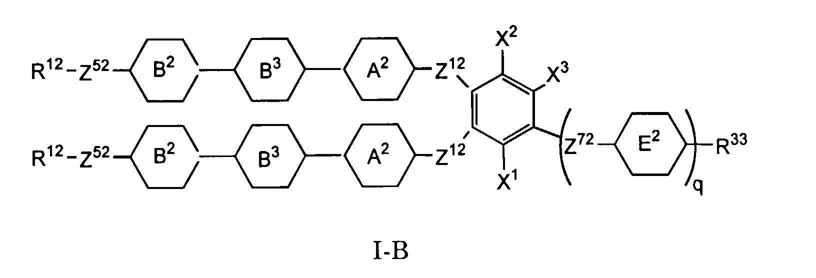 wherein
A², B², B³ and E² independently of one another signify 1,4-phenylene optionally substituted with fluorine or trans-1,4-cyclohexylene;
Z¹² signifies -(CH₂)₂COO-, -(CH₂)_{s'}COO- or -(CH₂)_{s'}O-;
Z⁵² signifies -(CH₂)_{s'}-, -(CH₂)_{s'}O-, -(CH₂)S'COO- or -(CH₂)_{s'}OOC-;
Z⁷² signifies -CH₂O- or -COO-;
s' signifies a whole number of 3 to 12;
R¹² signifies CH₂=CH-COO-, CH₂=C(CH₃)-COO-, CH₂=CH-O- or 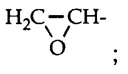
R³³ signifies halogen, cyano or alkoxycarbonyl;
q signifies 0 or 1; and
X¹, X² and X³ have the significance given in claim 1.

8. Compounds according to claim 7 of formulae Id-g 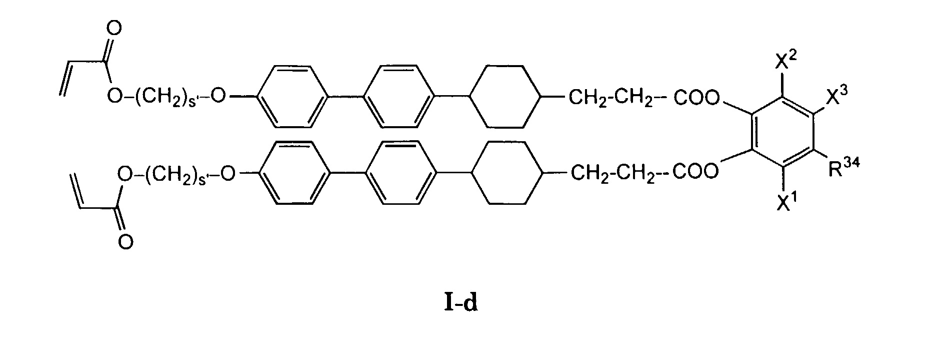 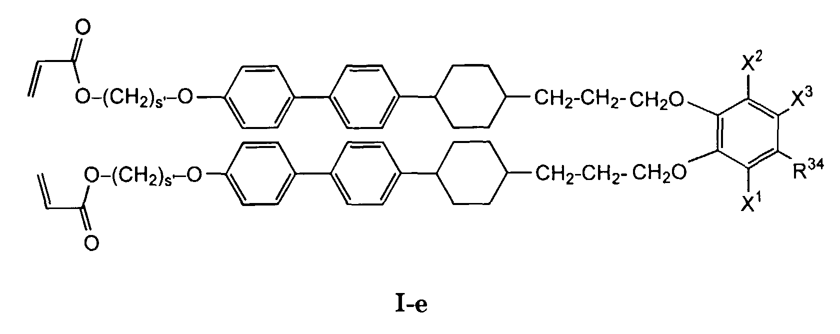 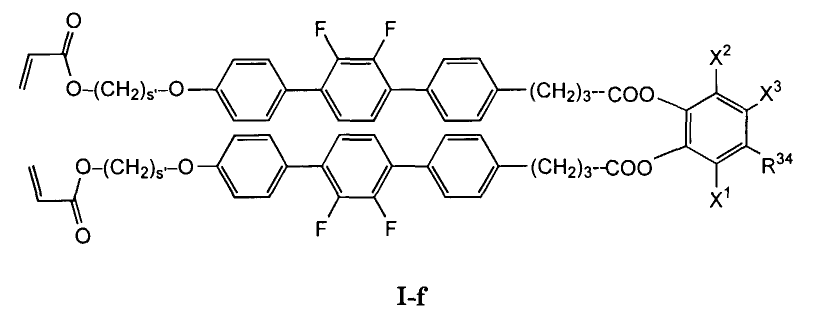 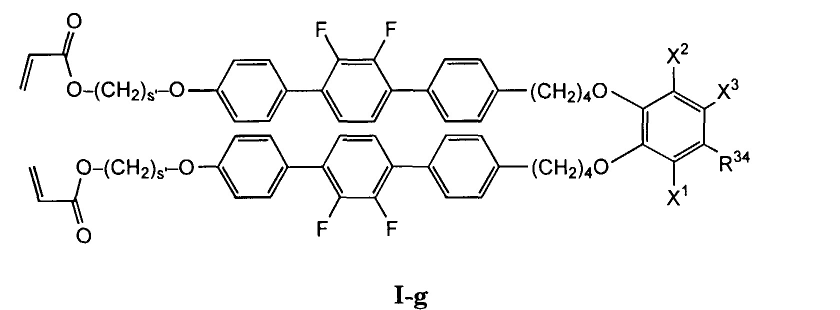 wherein R³⁴ signifies cyano or alkoxycarbonyl; X¹, X² and X³ signify hydrogen; and s' has the significance given in claim 7.

9. Cross-linkable liquid crystalline mixtures consisting of at least 2 components, wherein at least one component is a compound of formula I defined in claim 1.

10. Cross-linkable liquid crystalline mixtures according to claim 9, characterised in that they comprise, in addition to one or more compounds of formula I, one or more compounds from the group of the formulae 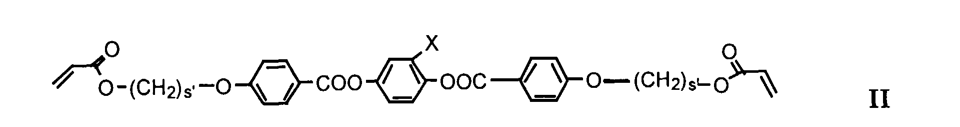 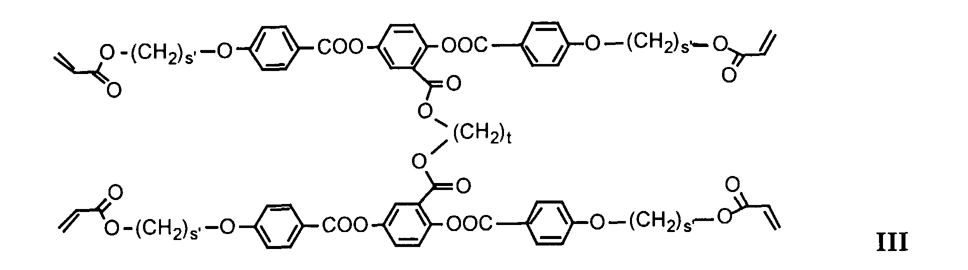 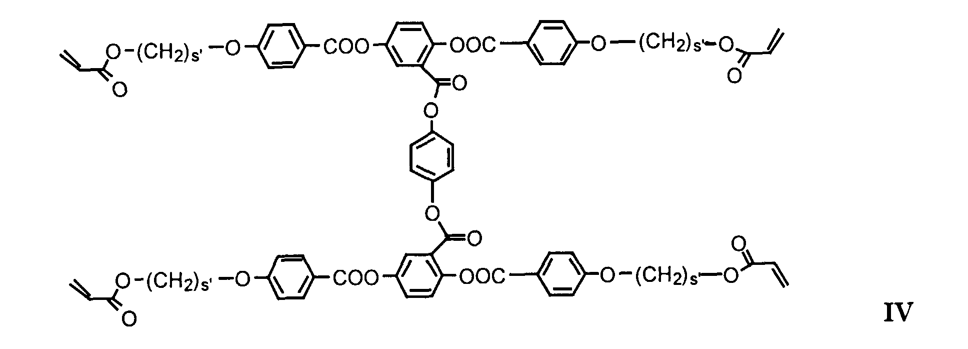 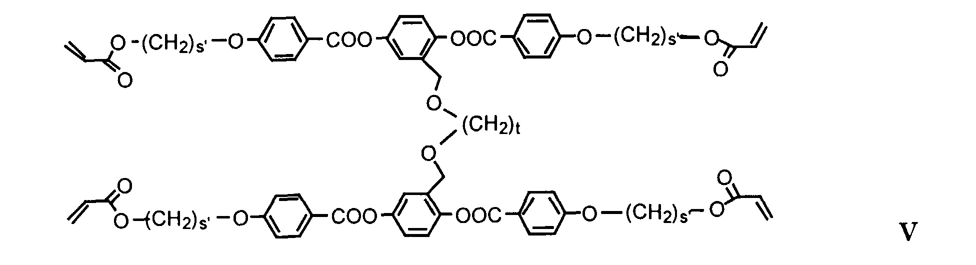 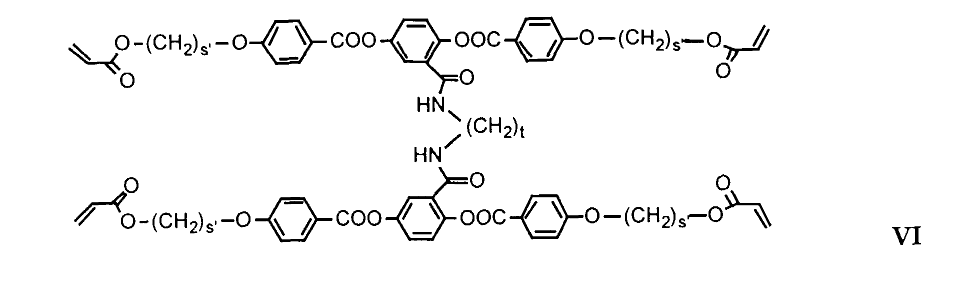 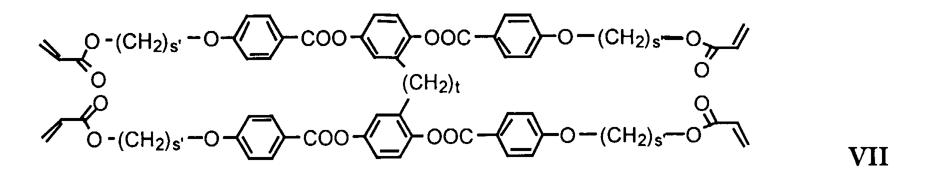 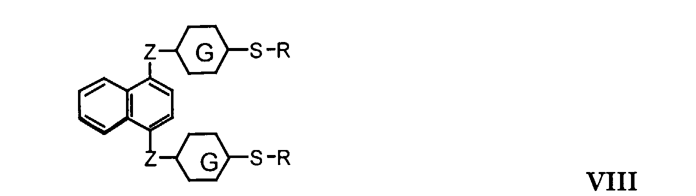 and 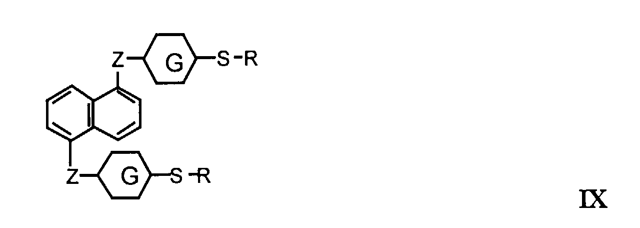 wherein
X signifies hydrogen, fluorine, chlorine, bromine or methyl;
s' signifies a whole number of 3 to 12;
t signifies a whole number of 2 to 12;
Z signifies -OCH₂- or -OOC-;
G signifies 1,4-phenylene or 2- or 3-fluoro-1,4-phenylene;
S -(CH₂)_{s'}- or -(CH₂)_{s'}O-; and
R signifies CH₂=CH-COO-, CH₂=C(CH₃)-COO-, CH₂=CH-O- or

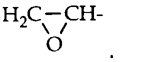

11. The use of compounds according to any one of claims 1 to 8 in their cross-linked state for optical components.

12. The use of cross-linkable liquid crystalline mixtures according to claim 9 or 10 in their cross-linked state for optical components.

## Revendications

1. Composé de formule générale I 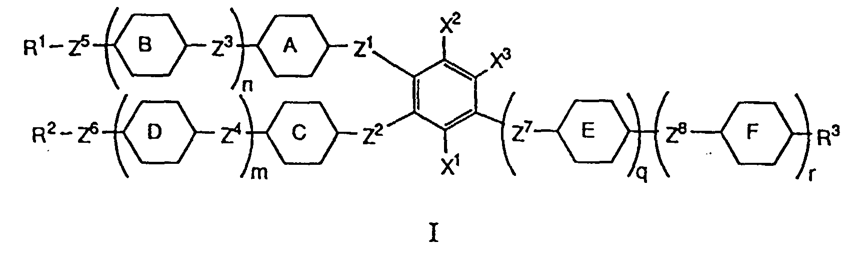 dans laquelle
les cycles A à F représentent chacun un groupe pyridin-2,5-diyle, pyrimidin-2,5-diyle, trans-1,4-cyclohexylène, trans-1,3-dioxan-2,5-diyle ou indépendamment les uns des autres un groupe 1,4-phénylène éventuellement substitué par un atome d'halogène, un groupe méthyle et/ou cyano;
Z¹, Z² et Z⁷ représentent indépendamment les uns des autres un groupe -CH₂-(CH₂)ₛ-, -(CH₂)ₛO-, -O-(CH₂)ₛ, -COO-, -OOC-, -(CH₂)ₛCOO- ou -(CH₂)ₛOOC-;
Z³, Z⁴ et Z⁸ représentent indépendamment les uns des autres une simple liaison, un groupe -CH₂CH₂-, -CH₂O-, -OCH₂-, -COO-, -OOC-, -(CH₂)₄-, -O(CH₂)₃- ou -(CH₂)₃O-;
Z⁵ et Z⁶ représentent un groupe -(CY₂)ₛ-, -O(CY₂)ₛ-, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂)CH₂O-, ou -NHCH₂(Si[(CH₃)₂]O)ₛSi[(CH₃)₂]CH₂NH-;
Y représente un atome d'hydrogène ou de fluor;
m, n, q et r représentent indépendamment les uns des autres 0, 1 ou 2;
s représente un nombre entier de 1 à 16;
R¹ et R² représentent des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 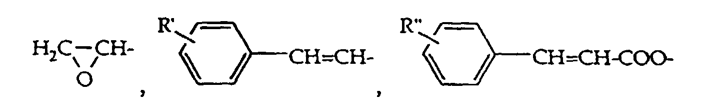
Ph représente le groupe phényle;
R' représente un groupe alkyle inférieur;
R" représente un groupe méthyle, méthoxy, cyano ou un atome d'halogène, avec la condition que R¹-Z⁵ et R²-Z⁶ ne contiennent pas de groupes -O-O- ni -N-O-;
R³ représente un atome d'hydrogène, d'halogène, un groupe cyano ou un groupe alkyle, alcoxy, alcoxycarbonyle ou alcanoyloxy éventuellement substitué par un groupe méthoxy, cyano et/ou un atome d'halogène; et
X¹, X² et X³ représentent indépendamment les uns des autres un atome d'hydrogène, d'halogène, un groupe cyano ou alkyle inférieur,
formule dans laquelle pour m = 2 et/ou n = 2 les deux cycles B et D peuvent être identiques ou différents.

2. Composé selon la revendication 1 de formule I 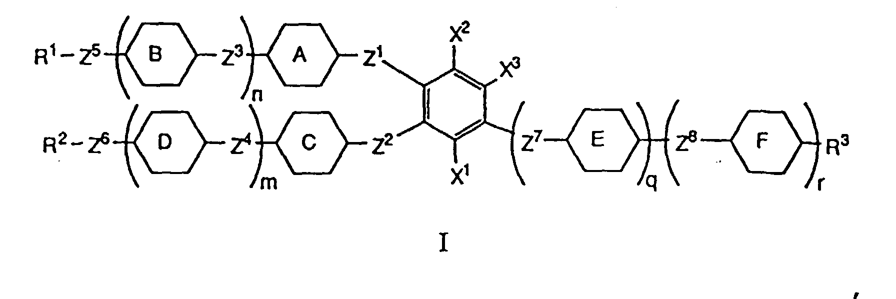 dans laquelle
les cycles A à F représentent chacun un groupe pyridin-2,5-diyle, pyrimidin-2,5-diyle, trans-1,4-cyclohexylène, trans-1,3-dioxan-2,5-diyle ou indépendamment les uns des autres un groupe 1,4-phénylène éventuellement substitué par un atome d'halogène, un groupe méthyle et/ou cyano;
Z¹, Z² et Z⁷ représentent indépendamment les uns des autres un groupe -CH₂-CH₂-, -CH₂-O-, -COO-, -OOC-,-(CH₂)₄- ou -(CH₂)₃O-;
Z³, Z⁴ et Z⁸ représentent indépendamment les uns des autres une simple liaison, un groupe -CH₂CH₂-, -CH₂O-, -OCH₂-, -COO-, -OOC-, -(CH₂)₄-, -O(CH₂)₃- ou -(CH₂)₃O-;
Z⁵ et Z⁶ représentent un groupe -(CY₂)ₛ, -O(CY₂)ₛ, -(CY₂)ₛO-, -(CY₂)ₛCOO-, -(CY₂)ₛOOC-, -(Si[(CH₃)₂]O)ₛ-, -OCH₂(Si[(CH₃)₂]O)ₛSi[(CH₃)₂]CH₂O-, ou -NHCH₂(Si[(CH₃)₂]O)ₛSi[(CH₃)₂]CH₂NH-;
Y représente un atome d'hydrogène ou de fluor;
m, n, q et r représentent indépendamment les uns des autres 0, ou 1;
s représente un nombre entier allant de 1 à 16; et
R¹ et R² représentent des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 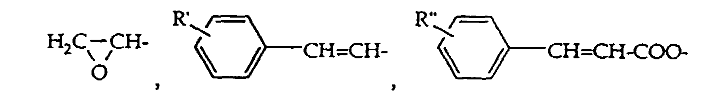
Ph représente le groupe phényle;
R' représente un groupe alkyle inférieur;
R" représente un groupe méthyle, méthoxy, cyano ou un atome d'halogène, avec la condition que R¹-Z⁵ et R²-Z⁶ ne contiennent pas de groupes -O-O- ni -N-O-;
R³ représente un atome d'hydrogène, d'halogène, un groupe cyano, ou un groupe alkyle, alcoxy ou alcanoyloxy éventuellement substitué par un groupe méthoxy, cyano et/ou un atome d'halogène; et
X¹, X² et X³ représentent indépendamment un atome d'hydrogène, d'halogène, un groupe cyano ou alkyle inférieur.

3. Composé selon la revendication 1 ou 2, dans lequel les groupes réticulables R¹ et R² représentent indépendamment l'un de l'autre un groupe CH₂=CH-COO-, CH₂=C(CH₃)-COO-, CH₂=CH-O-,

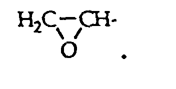

4. Composé selon l'une des revendications 1 à 3, dans lequel X¹, X² et X³ représentent un atome d'hydrogène, de fluor, de chlore, un groupe cyano et méthyle.

5. Composé selon l'une des revendications 1 à 4 de formule générale I-A 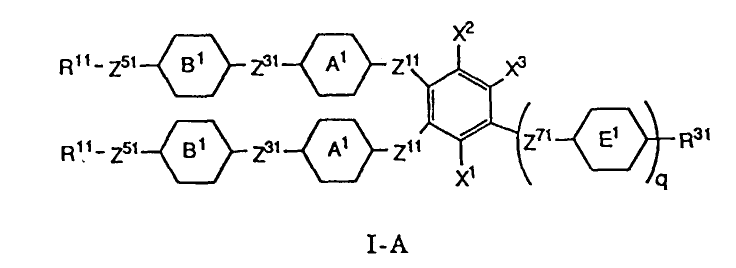 dans laquelle
A¹, B¹ et E¹ représentent indépendamment les uns des autres un groupe 1,4-phénylène éventuellement substitué avec du fluor, pyridin-2,5-diyle, pyrimidin-2,5-diyle ou trans-1,4-cyclohexylène;
Z¹¹ représente un groupe -CH₂CH₂-, -CH₂O-, -COO-, -OOC-, -(CH₂)₄- ou -(CH₂)₃O-;
Z³¹ représente une simple liaison, un groupe -CH₂O-, -COO- ou -OOC-;
Z⁵¹ représente un groupe -(CH₂)_{s'}-, -(CH₂)_{s'}O-, -(CH₂)_{s'}COO- ou -(CH₂)_{s'}OOC-;
Z⁷¹ représente un groupe -CH₂O- ou -COO-;
s' représente un nombre entier allant de 3 à 12;
R¹¹ représente CH₂=CH-COO-, CH₂=C(CH₃)-COO-, CH₂=CH-O-, 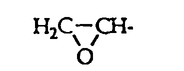
R³¹ représente un atome d'halogène, un groupe cyano, alkyle ou alcoxycarbonyle inférieur;
q représente 0 ou 1 et
X¹, X² et X³ ont la signification indiquée à la revendication 1.

6. Composés selon la revendication 5 de formules I-a à I-c 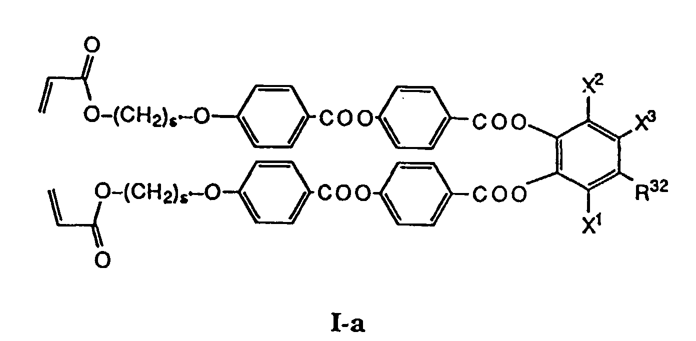 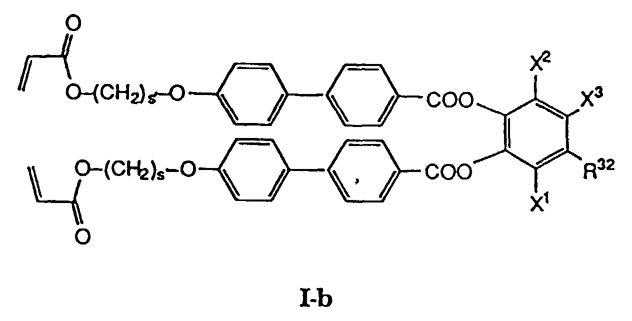 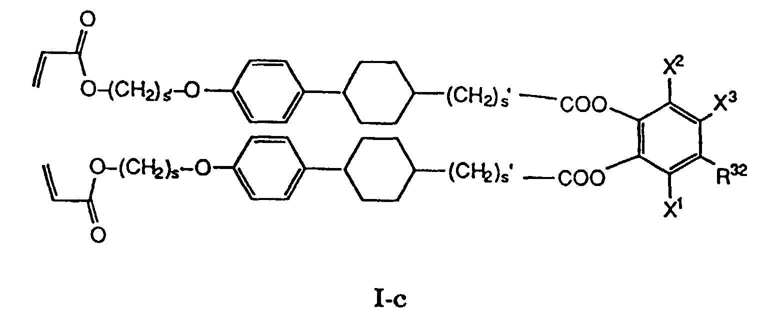 dans lesquelles R³² représente un groupe cyano ou alcoxycarbonyle; X¹, X² et X³ représentent un atome d'hydrogène; et s' a la signification indiquée à la revendication 5.

7. Composé selon l'une des revendications 1 à 4 de formule générale I-B 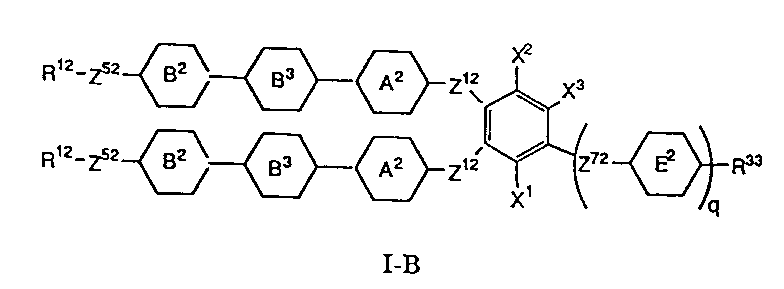 dans laquelle
A², B², B³ et E² représentent indépendamment les uns des autres un groupe 1,4-phénylène ou trans-1,4-cyclohexylène éventuellement substitué avec du fluor;
Z¹² représente un groupe -(CH₂)₂COO-, -(CH₂)_{s'}COO- ou -(CH₂)_{s'}O-;
Z⁵² représente un groupe -(CH₂)_{s'}-,-(CH₂)_{s'}O-, -(CH₂)_{s'}COO- ou -(CH₂)_{s'}OOC-;
Z⁷² représente un groupe -CH₂O- ou -COO-;
s' représente un nombre entier allant de 3 à 12;
R¹² représente CH₂=CH-COO-, CH₂=C(CH₃)-COO-, CH₂=CH-O-, 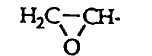
R³³ représente un atome d'halogène, un groupe cyano ou alcoxycarbonyle;
q représente 0 ou 1 et
X¹, X² et X³ ont la signification indiquée à la revendication 1.

8. Composés selon la revendication 7 de formules I-d à I-g 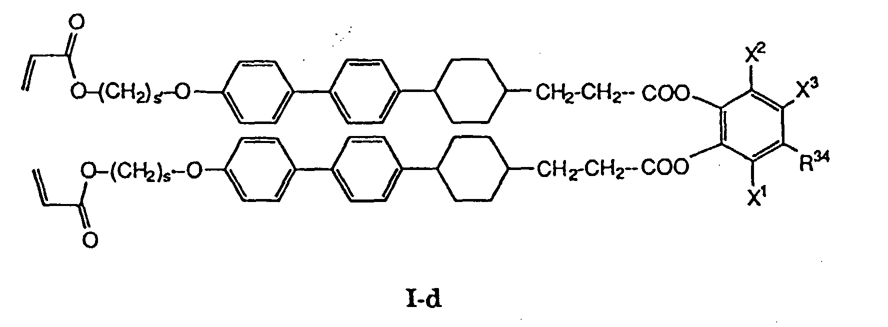 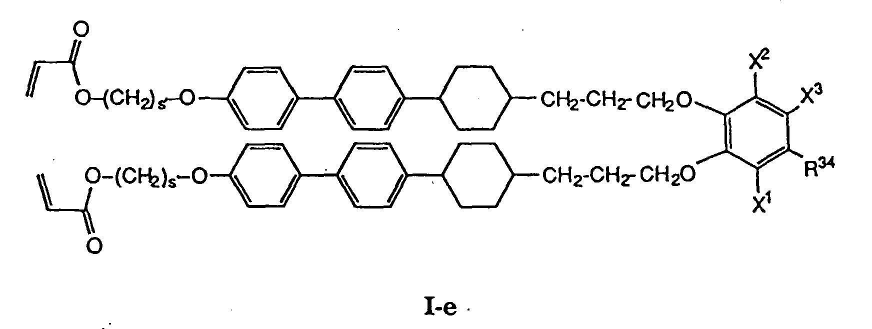 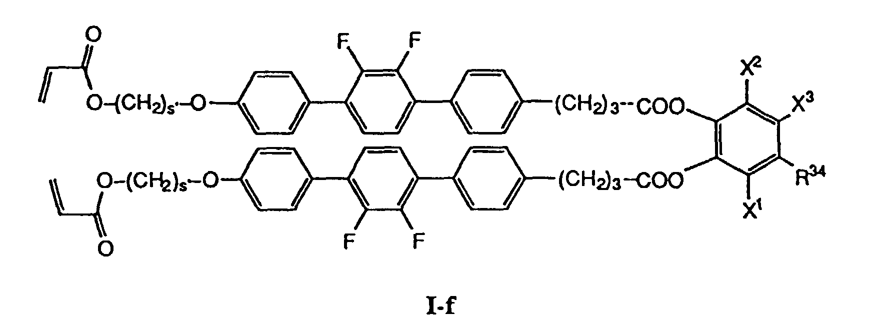 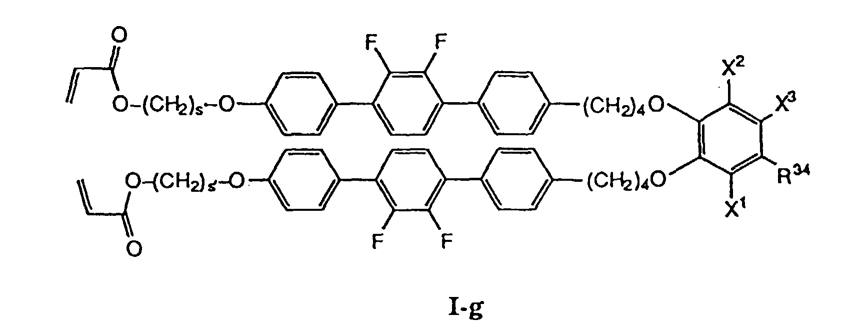 dans lesquelles R³⁴ représente un groupe cyano ou alcoxycarbonyle; X¹, X² et X³ représentent un atome d'hydrogène; et s' a la signification indiquée à la revendication 7.

9. Mélange réticulable pour cristaux liquides constitué d'au moins 2 composants, parmi lesquels au moins un composant est un composé de formule I définie à la revendication 1.

10. Mélange réticulable pour cristaux liquides selon la revendication 9, caractérisé en ce qu'il contient, en plus d'un ou de plusieurs composés de formule I, un ou plusieurs composés choisis dans le groupe de formules 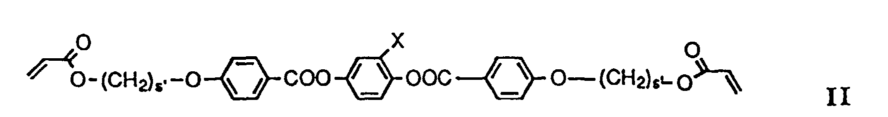 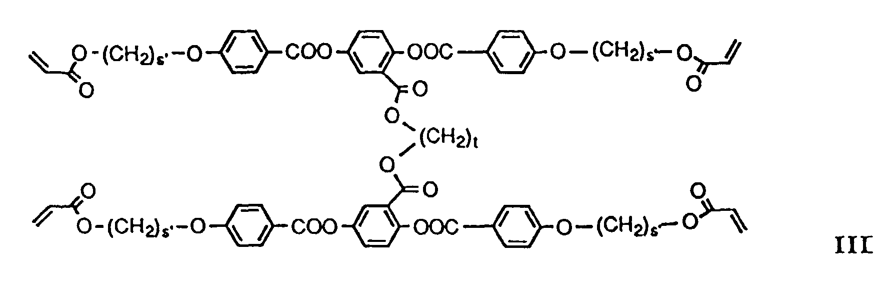 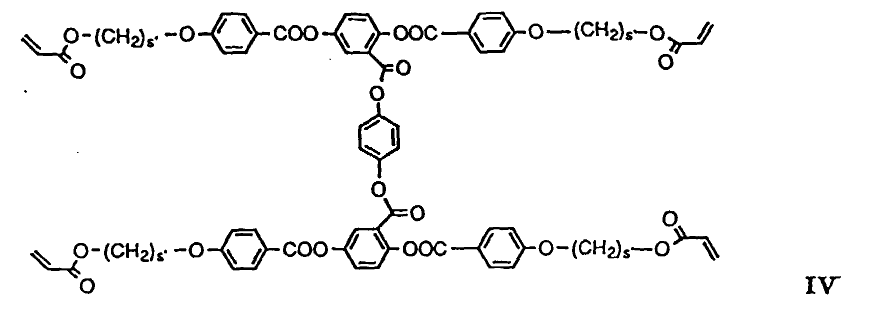 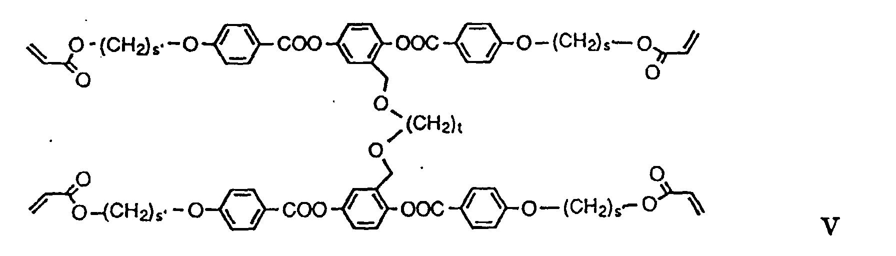 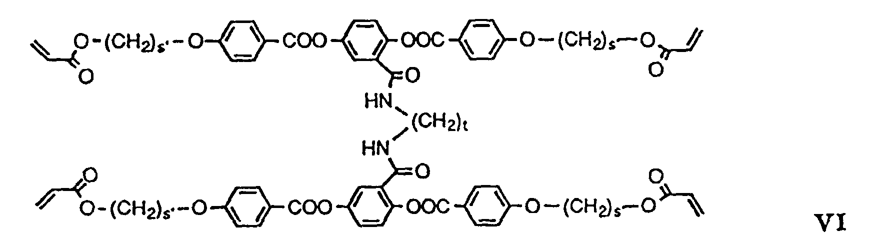 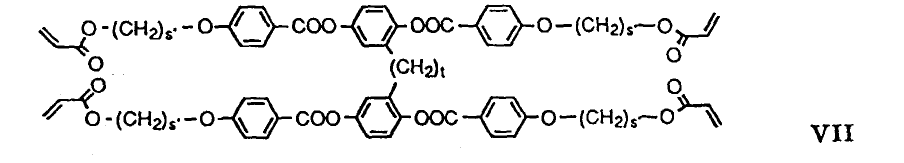 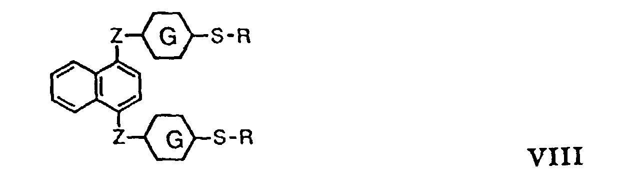 et 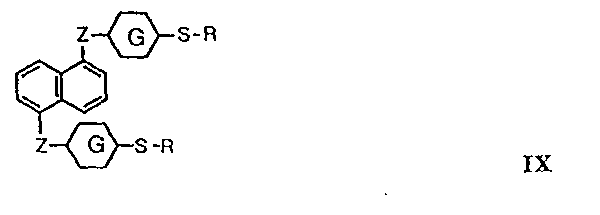 dans lesquelles
X représente un atome d'hydrogène, de fluor, de chlore, de brome ou un groupe méthyle;
s' représente un nombre entier allant de 3 à 12;
t représente un nombre entier allant de 2 à 12;
Z représente un groupe -OCH₂- ou -OOC-;
G représente un groupe 1,4-phénylène ou 2- ou 3-fluoro-1,4-phénylène;
S représente un groupe -(CH₂)_{s'}- ou -(CH₂)_{s'}O-; et
R représente un groupe CH₂=CH-COO-, CH₂=C(CH₃)-COO-, CH₂=CH-O-,

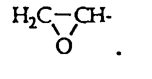

11. Utilisation de composés selon l'une des revendications 1 à 8 dans leur état réticulé pour des composants optiques.

12. Utilisation de compositions à cristaux liquides réticulables selon l'une des revendications 9 ou 10 dans leur état réticulé pour des composants optiques.
